(19) **Europäisches Patentamt / European Patent Office / Office européen des brevets**

(11) **EP 4 491 206 A1**

(12) # EUROPEAN PATENT APPLICATION

(43) Date of publication:
**15.01.2025 Bulletin 2025/03**

(21) Application number: **24186419.8**

(22) Date of filing: **04.07.2024**

(51) International Patent Classification (IPC):
**A61M 5/142** (2006.01)     **A61M 5/168** (2006.01)
**A61M 5/172** (2006.01)     **G16H 20/17** (2018.01)

(52) Cooperative Patent Classification (CPC):
**A61M 5/14216; A61M 5/1684; A61M 5/172;**
**G16H 20/17;** A61M 5/14244; A61M 5/14566;
A61M 2005/14208; A61M 2205/50

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **13.07.2023 CN 202310857662**

(71) Applicant: **Phray Technology Co., Ltd.**
**Zhengzhou Henan (CN)**

(72) Inventors:
• **Wang, Leijie**
  **Zhengzhou (CN)**
• **Zhang, Hongmei**
  **Zhengzhou (CN)**

• **Wang, Yongxin**
  **Zhengzhou (CN)**
• **Fang, Jucai**
  **Zhengzhou (CN)**
• **Dong, Ting**
  **Zhengzhou (CN)**
• **Yan, Jun**
  **Zhengzhou (CN)**
• **Zhang, Dandan**
  **Zhengzhou (CN)**
• **Xie, Zhuobin**
  **Zhengzhou (CN)**
• **Ming, Wuyi**
  **Zhengzhou (CN)**

(74) Representative: **Zaboliene, Reda**
**Metida**
**Business center Vertas**
**Gyneju str. 16**
**01109 Vilnius (LT)**

(54) **INTELLIGENT CONTROL METHOD FOR REFILL-TYPE SCREW INSULIN INJECTION PUMP**

(57)     Provided is an intelligent control method for a refill-type screw insulin injection pump, including the following steps: starting the refill-type screw insulin injection pump, and filling prefabricated liquid insulin medicine into the refill injection mechanism; operating the key operation module to enable the intelligent control module to control the power device to work; when the primary telescopic screw mechanism extends to a limit, the sensor assembly transmits a signal to the intelligent control module, and the intelligent control module controls a rotational speed of the power device to be slowed down to reduce impact force of the primary telescopic screw mechanism; the refill injection mechanism performs the injection of liquid insulin medicine; and repeating the above steps, and refilling liquid insulin medicine to prepare for the next injection upon completion of the injection of liquid insulin medicine.

FIG. 1

**Description**

**TECHNICAL FIELD**

The present disclosure belongs to the technical field of insulin injection, and particularly relates to an intelligent control method for a refill-type screw insulin injection pump.

**BACKGROUND**

An insulin pump is composed of an injection pump, a small syringe, and an infusion tube connected thereto, with a basic purpose of injecting insulin according to a required dosage. However, an injection amount of insulin each time is very small, therefore, how to control the injection amount with high precision while ensuring a small size of the insulin pump becomes a pressing challenge that needs to be addressed.

Search discovered some reports on relevant patent documents in the existing literature, including:

1. An invention patent with the authorized publication number CN108159530 A, titled *Integrated Assembly Structure of Driving Rod and Motor for Insulin Pump,* belongs to the technical field of medical devices. In the patent, an insulin pump adopts an integrated assembly structure of a driving rod and a motor, both of the driving rod and the motor are fixed on the same fixed base to form an entirety, which ensures a concentricity of the driving rod and the motor, such that the driving rod can be smoothly extended and retracted under the driving of the motor, consistent guide is maximized, resistance is reduced, and a service life of a driving mechanism is guaranteed.

2. An invention patent with the authorized publication number CN109529154 A, titled *New Type of Multi-Stage Immersed Cylinder Insulin Pump Propulsion Mechanism,* belongs to the technical field of medical devices. The patent adopts a multi-stage immersed cylinder insulin pump propulsion mechanism, and traditional square guide grooves are replaced by setting a main guide rail, an auxiliary guide rail, and a main-auxiliary guide rod, thereby avoiding the problem that the traditional square guide grooves pose high processing precision requirements, and avoiding the problem that the insulin pump goes wrong caused by jamming of the insulin pump in the propulsion and returning process due to low processing precision.

The above two patents only address the effectiveness and structural durability of the driving mechanism, but do not involve the improvement of injection precision and the miniaturization of the pump itself, which are exactly the problems that the future precision medicine needs to be urgently solved. Medical devices capable of providing high-precision medicine delivery devices with excellent user experience are a mainstream direction for the future development of insulin injection pumps.

**SUMMARY**

An objective of the present disclosure is to provide an intelligent control method for a refill-type screw insulin injection pump. Through the present disclosure, an injection amount of liquid insulin medicine can be accurately controlled, high-precision drug delivery can be achieved, an overall size of the refill-type screw insulin injection pump is significantly reduced, a rotational torque and speed of a power motor can be controlled in an intelligent manner, impact force is reduced, consumption of electric energy is reduced, and an anti-locking function is realized. In order to achieve the above objective, the present disclosure adopts the following technical solution:

an intelligent control method for a refill-type screw insulin injection pump, the refill-type screw insulin injection pump includes a housing, a refill injection mechanism, a primary telescopic screw mechanism, a secondary telescopic screw mechanism, a power device, a power supply module and an intelligent control module;

the refill injection mechanism, the primary telescopic screw mechanism and the secondary telescopic screw mechanism are all coaxially and vertically disposed on a left side portion inside the housing, the power device, the power supply module and the intelligent control module are all disposed on a right side portion inside the housing, the power device is in transmission connection with the primary telescopic screw mechanism, the power device drives the primary telescopic screw mechanism to telescope, the secondary telescopic screw mechanism is installed inside the primary telescopic screw mechanism and is in transmission connection with the primary telescopic screw mechanism, the primary telescopic screw mechanism drives the secondary telescopic screw mechanism to telescope, the refill injection mechanism is disposed on an upper portion of the secondary telescopic screw mechanism and is connected to an telescopic end of the secondary telescopic screw mechanism, a key operation module is disposed on the housing, a sensor assembly is disposed on the primary telescopic screw mechanism, the power supply module supplies power to the power device and the intelligent control module, and the intelligent control module is in signal connection with the power device, the key operation module and the sensor assembly, respectively;

a bottom of the housing is open and fixedly connected to a bottom cover, an injection cartridge similar to a pen holder structure is integrally formed on a left side of an interior of the housing in a vertical direction, an upper end of the injection cartridge penetrates upwards through a top of the housing upwards, a lower end of the injection cartridge extends downwards to a lower left portion of the housing, an inner electrical cavity is formed inside a lower right portion of the housing, the power supply module and the intelligent control module are disposed inside the inner electrical cavity, and the power device is disposed inside the upper right portion of the housing; and

the intelligent control method includes the following steps:

step (I) starting the refill-type screw insulin injection pump, operating the key operation module to enable the intelligent control module to control the power device to work, and to enable the power device to drive the primary telescopic screw mechanism and the secondary telescopic screw mechanism to telescope, and allowing prefabricated liquid insulin medicine to be filled into the refill injection mechanism;

step (II) controlling the power device to work through the intelligent control module, and driving the primary telescopic screw mechanism to extend first through the power device; when the primary telescopic screw mechanism extends to a limit, the sensor assembly transmits a signal to the intelligent control module, and the intelligent control module controls a rotational speed of the power device to be slowed down to reduce impact force of the primary telescopic screw mechanism, the secondary telescopic screw mechanism then starts to extend until it extends to a maximum limit position, and the intelligent control module controls the power device to stop; and in the extension process of the primary telescopic screw mechanism and the secondary telescopic screw mechanism, the refill injection mechanism performs the injection of liquid insulin medicine; and

step (III) repeating the step (I) upon completion of the injection of liquid insulin medicine, and refilling liquid insulin medicine to prepare for a next injection.

Preferably, the refill injection mechanism includes a refill and a refill piston, the refill is coaxially and fixedly embedded inside the injection cartridge, an outer circle of the refill is in close contact with an inner circle of the injection cartridge, a lower end of the refill is open, the lower end of the refill is located inside the injection cartridge and is close to the lower end of the injection cartridge, a T-shaped hollow cylindrical head with a large top but a small bottom is integrally formed on an upper top of the refill, a maximum circumferential diameter of the T-shaped hollow cylindrical head is smaller than a diameter of the refill, a lower end of the T-shaped hollow cylindrical head is connected to the upper top of the refill through a truncated cone tube with a narrow top but a wide bottom; and the upper end of the injection cartridge is coaxially and threadedly connected with a needle cap, the T-shaped hollow cylindrical head is embedded inside the needle cap, a cylindrical protrusion is coaxially and integrally formed in a center of a top of the needle cap, a first central hole is vertically formed in a center of the cylindrical protrusion, an injection hose is coaxially and fixedly embedded inside the first central hole, an upper end of the injection hose extends out from an upper end of the cylindrical protrusion and is connected to an injection needle, a second central hole coaxial with the first central hole is formed in a center of the needle cap, an inner diameter of the second central hole is smaller than an inner diameter of the first central hole, an upper end of the second central hole is connected to a lower end of the first central hole, a barrel is coaxially and fixedly embedded inside the second central hole, the barrel coaxially penetrates through the T-shaped hollow cylindrical head and extends into the T-shaped hollow cylindrical head, a lower end of the barrel is communicated with an interior of the refill, and the refill piston is coaxially sealed and slidably disposed inside the refill.

Preferably, the primary telescopic screw mechanism includes a primary fixing rod, a primary tubular screw, a driving cylinder and a fixing base, a circular convex edge is integrally formed on an outer edge of an upper surface of the fixing base, a third central hole is formed in a center of the fixing base, the primary fixing rod is coaxially disposed below the injection cartridge, an upper end of the primary fixing rod is located in a center of the lower end of the injection cartridge, a lower end of the primary fixing rod is fixedly connected to the third central hole, the primary tubular screw is coaxially sleeved outside the primary fixing rod, the driving cylinder is coaxially sleeved outside the primary tubular screw and is disposed between the injection cartridge and the fixing base, an upper end of the driving cylinder is rotationally connected to the lower end of the injection cartridge, a lower end of the driving cylinder is rotationally connected to the circular convex edge, an inner diameter of the driving cylinder is smaller than an inner diameter of the refill, first internal thread is formed on an inner circle of the upper end of the driving cylinder, first external thread in threaded fit with the first internal thread is axially formed in an outer ring of the primary tubular screw, second internal thread is formed on an inner ring of an upper end portion of the primary tubular screw, a lower end of the primary tubular screw is flush with the lower end of the driving cylinder and is higher than the upper surface of the fixing base, an upper end of the primary tubular screw is higher than the upper end of the driving cylinder, a limiting ring located above the upper end of the driving cylinder is fixedly sleeved on an outer circle of the upper end portion of the primary tubular screw, a first wedge-shaped clip protruding upwards is disposed on the upper end surface of the driving cylinder, and a second wedge-shaped clip protruding downwards is disposed on a lower end surface of the limiting ring; and when the primary tubular screw is retracted downwards inside the driving cylinder and moves downwards to a lower limit position, the first wedge-shaped clip is engaged with the second wedge-shaped clip, a third wedge-shaped clip protruding downwards is disposed on an upper end surface of the limiting ring, and a driving gear is fixedly sleeved on an outer circumference of the upper end of the driving cylinder;

the secondary telescopic screw mechanism includes a secondary fixing sleeve and a secondary tubular screw, the secondary fixing sleeve is coaxially and slidably sleeved on the primary fixing rod, the secondary tubular screw is coaxially and slidably sleeved on the secondary fixing sleeve and is located inside the primary tubular screw, second external thread in threaded fit with the second internal thread is axially formed in an outer ring of the secondary tubular screw, an upper end of the secondary fixing sleeve is flush with the upper end of the primary fixing rod, a lower end of the secondary fixing sleeve is flush with the lower end of the primary tubular screw, an upper end of the secondary tubular screw is higher than the upper end of the primary tubular screw and is fixedly connected to a limiting screw plunger located above the limiting ring, a diameter of the limiting screw plunger is smaller than the inner diameter of the refill, a top of the limiting screw plunger extends into the refill and is fixedly connected to a bottom of the refill piston, and a fourth wedge-shaped clip protruding downwards is disposed on an outer edge of a bottom of the limiting screw plunger; and when the secondary tubular screw is retracted downwards inside the primary tubular screw and moves downwards to a lower limit position, the fourth wedge-shaped clip is engaged with the third wedge-shaped clip, a lower end of the secondary tubular screw is higher than the lower end of the secondary fixing sleeve, a first limiting slot which is open on lower and outer sides is formed on an limiting block of the primary fixing rod in an axial direction, a first limiting block correspondingly and slidably clamped inside the first limiting slot is integrally formed on an inner wall of a lower end portion of the secondary fixing sleeve, a second limiting slot which is open on lower and outer sides is formed an outer wall of the secondary fixing sleeve in an axial direction, and a second limiting block correspondingly and slidably clamped inside the second limiting slot is integrally formed on an inner wall of a lower end portion of the secondary tubular screw; and
the first internal thread, the first external thread, the second internal thread and the second external thread are trapezoidal threads with the same thread pitch.

Preferably, the power device includes a power motor, the power motor fixedly mounted on an upper right portion of the housing, an output shaft of the power motor is vertically disposed on a lower side of the power motor, a motor gear is coaxially and fixedly mounted on a lower end of the output shaft of the power motor, the motor gear is located on a right side of the driving gear and is in meshing transmission connection with the driving gear, the power module is electrically connected to the power motor, and the intelligent control module is in signal connection with the power motor;

the sensor assembly includes a position sensor, the position sensor is disposed on a lower side face of a trapezoidal thread at a lower end of the first internal thread, a wire harness channel is axially formed on a cylinder body of the driving cylinder corresponding to the position sensor, a mounting hole with a diameter greater than that of the wire harness channel is coaxially formed on a lower end of the wire harness channel, a lower port of the mounting hole is formed on a lower end surface of the driving cylinder, an elastic conductor is fixedly disposed inside an upper portion of the mounting hole, one first signal wire runs through the wire harness channel, an upper end of the first signal wire is connected to the position sensor, a lower end of the first signal wire is connected to the elastic conductor, a lower end of the elastic conductor is fixedly connected to a conductive rolling ball located at a lower portion inside the mounting hole, a lower side portion of the conductive rolling ball protrudes downwards to form a lower port of the mounting hole, a conductive sheet is disposed on the upper surface of the fixing base, the conductive sheet is in rolling contact with the conductive rolling ball in an up-down correspondence manner, and the conductive sheet is in signal connection with the intelligent control module through a second signal wire passing through a lower surface of the fixing base and is introduced into the inner electrical cavity;
the refill is made of transparent material, a camera with built-in light embedded in an inner wall of the injection cartridge is disposed in a middle position of an outer wall of the refill, the power module is electrically connected to the camera, and the intelligent control module is in signal connection with the camera; and
the intelligent control module is a microcomputer with an embedded processor, and the intelligent control module is further provided with a built-in soft limit of the secondary telescopic screw mechanism, where soft limit parameters thereof are determined according to geometric parameters, that is, thread pitches, total lengths and working lengths, of the primary tubular screw and the secondary tubular screw, the intelligent control module immediately controls the power motor to stop rotating when the second limiting block on the inner wall of the lower end portion of the secondary tubular screw slides upwards to an upper end of the second limiting slot and is retained, and the secondary tubular screw retracts downwards inside the primary tubular screw and moves downwards to the lower limit position.

Preferably, the step (I) is specifically as follows:

the intelligent control module is initialized when the power module is turned on, the injection needle is inserted into an insulin injection bottle, the key operation module is subjected to operation, the intelligent control module controls the power motor to rotate reversely, the output shaft of the power motor drives the driving cylinder to rotate reversely through the motor gear and the driving gear, the driving cylinder drives the primary tubular screw to retract downwards inside the driving cylinder through the first internal thread and the first external thread, the primary tubular screw then drives the secondary fixing sleeve and the secondary tubular screw to move downwards relative to the primary fixing rod, and the secondary tubular screw drives the limiting screw plunger and the refill piston to move downwards, such that the refill piston can draw the liquid insulin medicine in the insulin injection bottle into the refill through the injection needle, the injection hose and the barrel;

when the primary tubular screw retracts downwards inside the driving cylinder and moves downwards to the lower limit position, the second wedge-shaped clip is engaged with the first wedge-shaped clip, and the primary tubular screw cannot continue to move downwards, therefore, the driving cylinder drives the primary tubular screw to rotate in an opposite direction through the first wedge-shaped clip and the second wedge-shaped clip, the primary tubular screw and the secondary tubular screw rotate relative to each other, the primary tubular screw drives the secondary tubular screw to retracts downwards into the primary tubular screw through the second internal thread and the second external thread, the secondary tubular screw continues to drive the limiting screw plunger and the refill piston to move downwards, and the refill piston continues to draw the liquid insulin medicine in the insulin injection bottle into the refill through the injection needle, the injection hose and the barrel; and

when the secondary tubular screw retracts downwards into the primary tubular screw and moves downwards to the lower limit position, the fourth wedge-shaped clip is engaged with the third wedge-shaped clip, the refill is filled with the liquid insulin medicine, the filling operation of the liquid insulin medicine is completed, and a new injection needle head is replaced to prepare for the injection.

Preferably, the step (II) is specifically as follows:

the key operation module is subjected to operation, the intelligent control module controls the power motor to rotate forward, the output shaft of the power motor drives the motor gear to rotate, the motor gear is meshed with and drives the driving gear, the driving gear then drives the driving cylinder to rotate forward, the driving cylinder and the primary tubular screw rotate relative to each other, the driving cylinder drives the primary tubular screw to extend out from the driving cylinder through the first internal thread and the first external thread, the primary tubular screw then drives the secondary fixing sleeve and the secondary tubular screw to move upwards relative to the primary fixing rod, the first limiting block on the inner wall of the lower end portion of the secondary fixing sleeve slides upwards in the first limiting slot on the outer side wall of the primary fixing rod, the secondary tubular screw pushes the limiting screw plunger and the refill piston to move upwards, such that the refill piston pushes out the liquid insulin medicine in the refill and performs the injection through the barrel, the injection hose and the injection needle;

the intelligent control module acquires the signal from the position sensor, obtains information about the number of rotations of the driving cylinder, and obtains a distance that the primary tubular screw moves upwards according to the number of rotations of the driving cylinder; and

meanwhile, the intelligent control module processes the collected images through a pyramid YOLO7 algorithm to identify a position of the refill piston, an injected amount of liquid insulin medicine in the refill is then measured, the intelligent control module controls the power motor to change a rotational torque and speed, impact face between the primary tubular screw and the driving cylinder is reduced until the primary tubular screw moves upwards to an upper limit position; when the first limiting block slides upwards to an upper end of the first limiting slot and is retained, the primary tubular screw is in transmission connection with the driving cylinder to form an entirety, an movement form of the primary tubular screw changes from moving up and down in a vertical direction to be rotated together with the driving cylinder, the primary tubular screw and the secondary tubular screw rotate relative to each other, the primary tubular screw drives the secondary tubular screw to extend upwards and out from the primary tubular screw through the second internal thread and the second external thread, the secondary tubular screw moves upwards relative to the secondary fixing sleeve, the secondary tubular screw continues to push the limiting screw plunger and the refill piston to move upwards, and the refill piston continues to push out the liquid insulin medicine in the refill and is injected through the barrel, the injection hose and the injection needle; and when the second limiting block on the inner wall of the lower end of the secondary tubular screw slides upwards to the upper end of the second limiting slot and is retained, the secondary tubular screw moves upward to its upper limit position, the intelligent control module controls the power motor to stop rotating, and the whole injection process of the liquid insulin medicine is completed.

Preferably, the intelligent control module controls the power motor to change the rotational torque and speed through the following methods:

when the driving cylinder rotates forward to reach a first set number of times, the primary tubular screw moves upward to start to approach its upper limit position state, the intelligent control module measures through the camera that the injected amount of liquid insulin medicine in the refill is greater than one third or start to approach one half, and the intelligent control module controls driving current of the power motor to be increased to 125% of a standard state through the camera, improves the rotational torque of the power motor, and reduces the speed of the power motor to 80% of a standard state through PWM speed regulation;

when the driving cylinder rotates forward to reach a second set number of times, the primary tubular screw moves upward to continue to approach its upper limit position state, the intelligent control module recognizes through the camera that the refill piston moves further upwards, and measures that the injected amount of liquid insulin medicine in the refill further approaches one half, and the intelligent control module controls driving current of the power motor to be increased to 150% of a standard state through the camera, improves the rotational torque of the power motor, and reduces the speed of the power motor to 60% of a standard state through PWM speed regulation;

when the driving cylinder rotates forward to reach a third set number of times, the primary tubular screw moves upward to continue to approach its upper limit position state, the intelligent control module recognizes through the camera that the refill piston moves further upwards, and measures that the injected amount of liquid insulin medicine in the refill approaches one half, and the intelligent control module controls driving current of the power motor to be increased to 200% of a standard state through the camera, improves the rotational torque of the power motor, reduces the speed of the power motor to 20% of a standard state through PWM speed regulation, and further reduces the impact force of the primary tubular screw and the driving cylinder; and

when the driving cylinder rotates forward to reach a fourth set number of times, the primary tubular screw moves upward to reach its upper limit position state, the intelligent control module recognizes through the camera that the refill piston moves further upwards, and measures that the injected amount of liquid insulin medicine in the refill further reaches one half, and the intelligent control module controls driving current of the power motor to be increased to 300% of a standard state through the camera, improves the rotational torque of the power motor, and reduces the speed of the power motor to 5% of a standard state through PWM speed regulation.

Preferably, the pyramid YOLO7 algorithm includes the following steps:

obtaining an original input image with a resolution of w × h pixels captured by the camera;

performing rectangular window cropping on keyframes of the original input image, and denoting a starting point and an ending point of the rectangular window cropping as A($x_1$,$y_1$) and B ($x_2$,$y_2$);

acquiring the captured images once every 10 ms, and determining whether an image threshold is greater than 5 ml/h;

acquiring an image of which keyframes collected by the camera at a current moment are subjected to the rectangular window cropping and denoting as Image A when the image threshold is greater than 5 ml/h and a current injected amount is less than half of a capacity of the refill;

taking the current moment as the starting point to acquire images of which keyframes over the previous 500 ms and the previous 2 s are subjected to the rectangular window cropping, denoting as Images B and C, respectively; processing Images A, B and C respectively according to a preset target detection model to obtain target detection probabilities $P_A$, $P_B$ and $P_C$ of Images A, B and C;

obtaining maximum values of all dimensions of the target detection probabilities $_A$, $P_B$ and $P_C$, and obtaining an injection speed and an injection amount at the current moment according to the obtained maximum values and a maximum value determination principle; and

starting a new round of detection after completion of the current detection.

Preferably, the starting point A ($x_1$,$y_1$) and the ending point B($x_2$,$y_2$) of the rectangular window cropping are determined according to the following methods:

(1) historically detected data is analyzed to obtain coordinates of a maximum circumscribed rectangle area of a target object hotspot marked by the pyramid YOLO7 algorithm with a self-adaptive weight over the most recent 7 days, which are respectively denoted as: $S_1\{(x_{11},y_{11}), (x_{21},y_{21})\}$, $S_2\{(x_{12},y_{12}), (x_{22},y_{22})\}$, $S_3\{(x_{13},y_{13}), (x_{23},y_{23})\}\cdots S_n\{(x_{1n},y_{1n}), (x_{2n},y_{2n})\}$;

coordinates of an X direction and a Y direction of the starting point A ($x_1$, $y_1$) are expressed as Formulae (1) and (2) below:

$$x_1 = 0.8 * (x'_{11} + x'_{12} + x'_{13} + \cdots + x'_{1[n/2]})/[n/2] \quad (1);$$

$$y_1 = 0.8 * (y'_{11} + y'_{12} + y'_{13} + \cdots + y'_{1[n/2]})/[n/2] \quad (2);$$

where [n/2] is n/2 rounded down, and $x_1$, $y_1$ are both greater than and equal to 1; $x'_{11}, x'_{12}, x'_{13}\ldots\ldots x'_{1[n/2]}$, and $y'_{11}$, $y'_{12}, y'_{13}\ldots\ldots y'_{1[n/2]}$ are data series $x_{11}, x_{12}, x_{13},\ldots\ldots x_{1n}$ and $y_{11}, y_{12}, y_{13}, \ldots\ldots y_{1n}$ are arranged in ascending order respectively, and the first 50% of the data series are taken;

coordinates of an X direction and a Y direction of the ending point B ($x_2$,$y_2$) are expressed as Formulae (3) and (4) below:

$$x_2 = 0.8 * (x'_{21} + x'_{22} + x'_{23} + \cdots + x'_{2[n/2]})/[n/2] \quad (3);$$

$$y_2 = 0.8 * (y'_{21} + y'_{22} + y'_{23} + \cdots + y'_{2[n/2]})/[n/2] \quad (4);$$

where $x_2$ is less than and equals to 1280, $y_2$ is less than and equals to 720; $x'_{21}, x'_{22}, x'_{23}\ldots x'_{2[n/2]}$ and $y'_{21}, y'_{22}$, $y'_{23}\ldots y'_{2[n/2]}$ are data series $x_{21}, x_{22}, x_{23},\ldots x_{2n}$ and $y_{21}, y_{22}, y_{23}, \ldots y_{2n}$ are arranged in ascending order, and the first 50% of the data series are taken;

(2) when the cumulative number of days of historically data is less than 7 days, coordinates of the starting point A are set as (1, 1), and coordinates of the ending point B are set as (1280, 720).

Preferably, Image A is processed according to the preset target detection model, including the following steps:

performing target object detection on Image A, obtaining a maximum probability that the injection amount is less than or equals to 0, is greater than 0 but less than 1/2 and equals to 1 in Image A and denoting as P{a, b, c};

dividing Image A by two equal parts into four sub-images in length and width directions, obtaining maximum probabilities that the injection amount is less than or equals to 0, is greater than 0 but less than 1/2 and equals to 1 in the current sub-images, and denoting as $P_1$, $P_2$, $P_3$ and $P_4$; and selecting maximum values of $P_1$, $P_2$, $P_3$ and $P_4$ of the four sub-images in each dimension and denoting as $P_5${d, e, f};

dividing Image A by four equal parts into sixteen sub-images in length and width directions, obtaining maximum probabilities that the injection amount is less than or equals to 0, is greater than 0 but less than 1/2 and equals to 1 in the current sub-images and denoting as $P_{11}\ldots P_{14}\ldots P_{24}\ldots P_{44}$; and selecting maximum values of $P_{11}\ldots P_{14}\ldots P_{24}\ldots P_{44}$ of the sixteen sub-images in each dimension and denoting as $P_{55}${g, h, i}; obtaining the target detecting probability $P_A$ of Image A according to $P_5${ d, e, f}, $P_{55}${g, h, i} and Formula (5):

$$P_A = \left\{\frac{1}{3} \times (w_1 \times a + w_2 \times d + w_3 \times g), \frac{1}{3} \times (w_4 \times b + w_5 \times e + w_6 \times h), \frac{1}{3} \times (w_7 \times c + w_8 \times f + w_9 \times i)\right\}$$

(5);

where $w_1\ldots\ldots w_9$ are weights and are obtained by the following formulae:

$$W_1 = \frac{a}{a+b+c}, \quad W_2 = \frac{d}{d+e+f}, \quad W_3 = \frac{g}{g+h+i} \quad (6);$$

$$W_4 = \frac{b}{a+b+c}, \quad W_5 = \frac{e}{d+e+f}, \quad W_6 = \frac{h}{g+h+i} \quad (7);$$

$$W_7 = \frac{c}{a+b+c}, \quad W_8 = \frac{f}{d+e+f}, \quad W_9 = \frac{i}{g+h+i} \quad (8).$$

Preferably, the same steps are taken to process Images B and C to obtain the target detecting probabilities $P_B$ and Pc. Compared with the prior art, the present disclosure has prominent substantive features and notable progress. Specifically, the first internal thread is formed on the inner circle of the upper end of the driving cylinder, the first external thread in threaded fit with the first internal thread is axially formed in the outer ring of the primary tubular screw, the second internal thread is formed on the inner ring of the upper end portion of the primary tubular screw, the second external thread in threaded fit with the second internal thread is axially formed in the outer ring of the secondary tubular screw, and the first internal thread, the first external thread, the second internal thread and the second external thread are trapezoidal threads with the same thread pitch, therefore, one full rotation of the driving cylinder can make the primary tubular screw or the secondary tubular screw extend or retract by a length of one thread pitch, such that the injection amount of liquid insulin medicine can be accurately controlled, high-precision medicine delivery can be achieved, and an overall size of the refill-type screw insulin injection pump is significantly reduced, therefore, during the whole injection process of the liquid insulin medicine, the intelligent control module can calculate the number of rotations of the driving cylinder by acquiring the signal from the position sensor, the intelligent control module obtains a distance that the primary tubular screw moves upwards according to the number of rotations of the driving cylinder, and meanwhile, the intelligent control module processes images captured by the camera through a pyramid YOLO7 algorithm to identify a position of the refill piston, an injected amount of liquid insulin medicine in the refill is then measured, the intelligent control module controls the power motor to change a rotational torque and speed, impact face between the primary tubular screw and the driving cylinder is reduced, and consumption of electric energy is reduced; and the intelligent control module is further provided with a built-in soft limit of the secondary telescopic screw mechanism, where soft limit parameters thereof are determined according to geometric parameters, that is, thread pitches, total lengths and working lengths, of the primary tubular screw and the secondary tubular screw, therefore, the intelligent control module immediately controls the power motor to stop rotating when the second limiting block on the inner wall of the lower end portion of the secondary tubular screw slides upwards to an upper end of the second limiting slot and is retained, and the secondary tubular screw retracts downwards inside the primary tubular screw and moves downwards to the lower limit position, such that the secondary tubular screw is prevented from being tensioned or the secondary tubular screw enters the primary tubular screw and becomes too tight and is locked, and meanwhile, the power motor is prevented from being damaged due to too large torque of the output shaft caused by overloading of the power motor, and the effect of protecting the power motor, the primary tubular screw and the secondary tubular screw are achieved.

When the present disclosure is filled with the liquid insulin medicine, the power motor drives the driving cylinder to rotate reversely through the motor gear and the driving gear, such that the primary tubular screw is driven to retract downwards inside the driving cylinder; since a limiting ring located above the upper end of the driving cylinder is fixedly sleeved on an outer circle of the upper end portion of the primary tubular screw, a first wedge-shaped clip protruding upwards is disposed on an upper end surface of the driving cylinder, a second wedge-shaped clip protruding downwards is disposed on a lower end surface of the limiting ring, a third wedge-shaped clip protruding downwards is disposed on an upper end surface of the limiting ring, and a fourth wedge-shaped clip protruding downwards is disposed on an outer edge of a bottom of the limiting screw plunger; when the primary tubular screw retracts downwards inside the driving cylinder and moves downwards to the lower limit position, the second wedge-shaped clip is engaged with the first wedge-shaped clip, in which case, the primary tubular screw rotates reversely together with the driving cylinder to prevent the primary tubular screw from entering the driving cylinder deeply or tightly, resulting in being locked; the primary tubular screw and the secondary tubular screw rotate relative to each other, the primary tubular screw drives the secondary tubular screw to retracts downwards into the primary tubular screw through the second internal thread and the second external thread, and similarly when the secondary tubular screw retracts downwards inside the primary tubular screw and moves downwards to the lower limit position, the fourth wedge-shaped clip is engaged with the third wedge-shaped clip, the secondary tubular screw rod cannot continue to move downwards and retract into the primary tubular screw, in which case, the second wedge-shaped clip is engaged with the first wedge-shaped clip, and the fourth wedge-shaped clip is engaged with the third wedge-shaped clip, such that an anti-locking effect can be achieved in the structure.

To sum up, through the present disclosure, an injection amount of liquid insulin medicine can be accurately controlled, high-precision drug delivery can be achieved, an overall size of the refill-type screw insulin injection pump is significantly reduced, a rotational torque and speed of a power motor can be controlled in an intelligent manner, impact force is reduced, consumption of electric energy is reduced, and an anti-locking function is realized.

**BRIEF DESCRIPTION OF THE DRAWINGS**

Exemplary embodiments of the present disclosure may be more fully understood by reference to the following drawings. The accompanying drawings are used for providing further understanding of embodiments in the present disclosure, and constitute part of the specification, used, together with embodiments of the present disclosure, to explain the present disclosure and are not to be construed as limiting the present disclosure. In the drawings, like reference numerals generally represent like components or steps.

FIG. 1 is a schematic diagram of an initial state of the present disclosure that is filled with liquid insulin medicine and prepared for injection.
FIG. 2 is a schematic diagram of an intermediate state (a primary tubular screw moves upwards to an upper limit position) in a process of injecting liquid insulin medicine according to the present disclosure.

FIG. 3 is a schematic diagram upon completion of injection of insulin liquid medicine (a secondary tubular screw moves upwards to an upper limit position) according to the present disclosure.

FIG. 4 is a schematic diagram of an intermediate state (a primary tubular screw moves downwards to a lower limit position) in the process of refilling insulin liquid medicine according to the present disclosure.

FIG. 5 is a schematic diagram upon completion of refilling of insulin liquid medicine (a secondary tubular screw moves downwards to a lower limit position) according to the present disclosure.

FIG. 6 is a schematic diagram of a working state of a limiting ring when a primary tubular screw and a secondary tubular screw return to an initial state time upon completion of refilling of insulin liquid medicine according to the present disclosure.

FIG. 7 is a schematic elevation diagram of a position sensor, an elastic conductor, a first signal wire and a conductive rolling ball on a driving cylinder according to the present disclosure.

FIG. 8 is a schematic diagram of a pyramid YOLO7 algorithm of the present disclosure performing rectangular window cropping on keyframes of w× h pixels of an original input image.

FIG. 9 is a schematic diagram of a pyramid YOLO7 algorithm of the present disclosure performing YOLO7 target object detection on Image A.

FIG. 10 is a partial enlarged view of Area A in FIG. 1.

FIG. 11 is a partial enlarged view of Area B in FIG. 7.

Reference numerals in the accompanying drawings:

1. housing; 2. key operation module; 3. bottom cover; 4. injection cartridge; 5. inner electrical cavity; 6. refill; 7. refill piston; 8. T-shaped hollow cylindrical head; 9. truncated cone tube; 10.needle cap; 11. cylindrical protrusion; 12. injection hose; 13. barrel; 14. fixing base; 15. primary fixing rod; 16. primary tubular screw; 17. driving cylinder; 18. circular convex edge; 19. first internal thread; 20. first external thread; 21. second internal thread; 22. limiting ring, 23. first wedge-shaped clip; 24. second wedge-shaped clip; 25. third wedge-shaped clip; 26. driving gear' 27. driving cylinder; 28. secondary tubular screw; 29. second external thread; 30. limiting screw plunger; 31. fourth wedge-shaped clip; 32. first limiting slot; 33. first limiting block; 34. second limiting slot; 35. second limiting block; 36. power motor; 37. motor gear; 38. position sensor; 39. wire harness channel; 40. mounting hole; 41. elastic conductor; 42. first signal wire; 43. conductive rolling ball; and 44. conductive sheet.

## DETAILED DESCRIPTION OF EMBODIMENTS

Exemplary embodiments of the present disclosure will be described in more detail below with reference to the accompanying drawings. Although exemplary embodiments of the present disclosure are shown in the drawings, it should be understood that the present disclosure may be embodied in various forms and should not be limited by the embodiments set forth herein. Rather, these embodiments are provided for more thorough understanding of the present disclosure and to fully convey the scope of the present disclosure to those skilled in the art.

In the description of the present disclosure, it is to be noted that orientation or position relations indicated by the terms "central", "upper", "lower", "left", "right", "vertical", "horizontal", "inner", "outer", etc. are based on orientation or position relations shown in the accompanying drawings, and are merely for facilitating the description of the present disclosure and simplifying the description, rather than indicating or implying the device or element referred to must have a specific orientation or be constructed and operated in a specific orientation, and therefore will not be interpreted as limiting the present disclosure. In addition, the terms "first", "second" and "third" are for descriptive purposes only and should not be construed as indicating or implying relative importance.

In the description of the present disclosure, it should be noted that, unless otherwise explicitly specified and defined, the terms "mounting", "connecting" and "connection" should be understood in a broad sense, for example, they may be a fixed connection, a detachable connection, or an integrated connection; may be a mechanical connection, or an electrical connection; and may be a direct connection, or an indirect connection via an intermediate medium, or communication inside two elements. For those of ordinary skill in the art, the specific meanings of the above terms in the present disclosure may be understood according to specific circumstances.

Further, the technical features involved in different implementations of the present disclosure described below may be combined with one another as long as they do not constitute a conflict with one another.

An embodiment of the present disclosure provides an intelligent control method for a refill-type screw insulin injection pump, which is described below in conjunction with the accompanying drawings.

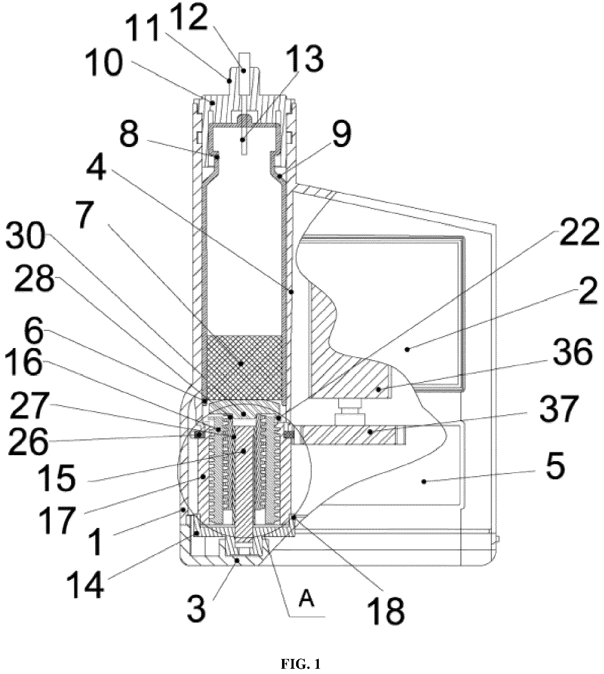

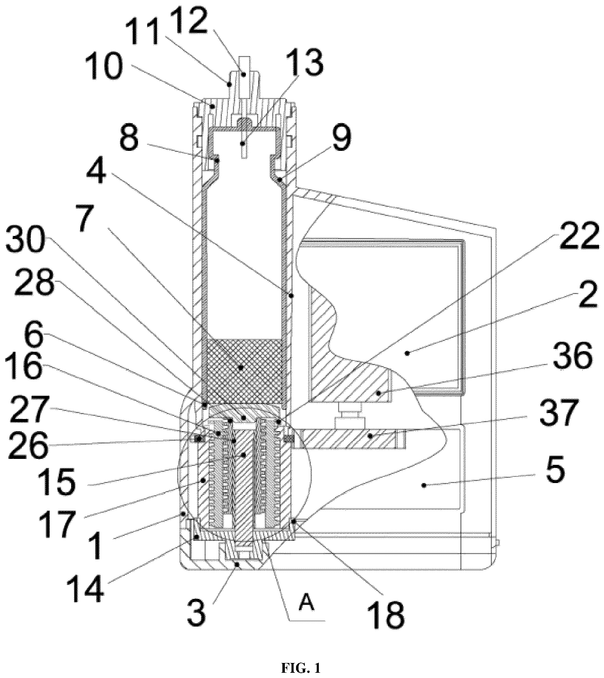

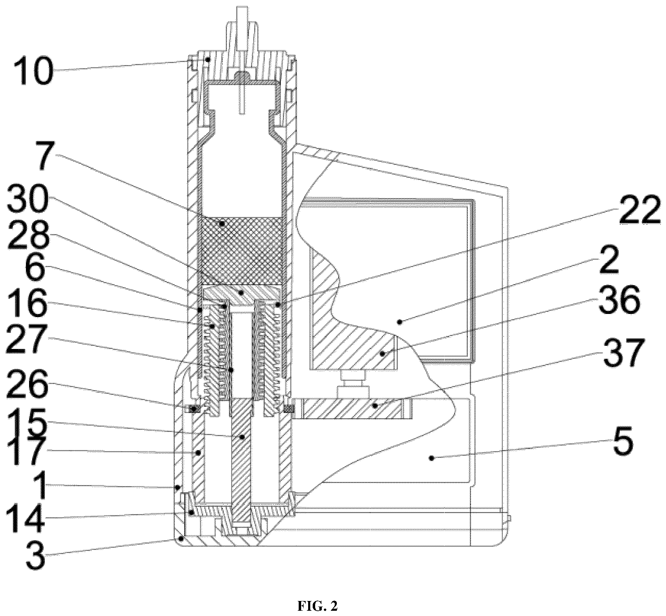

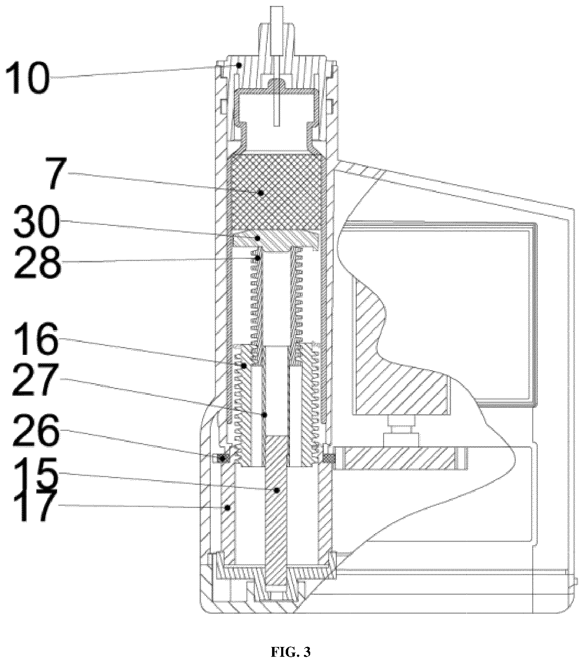

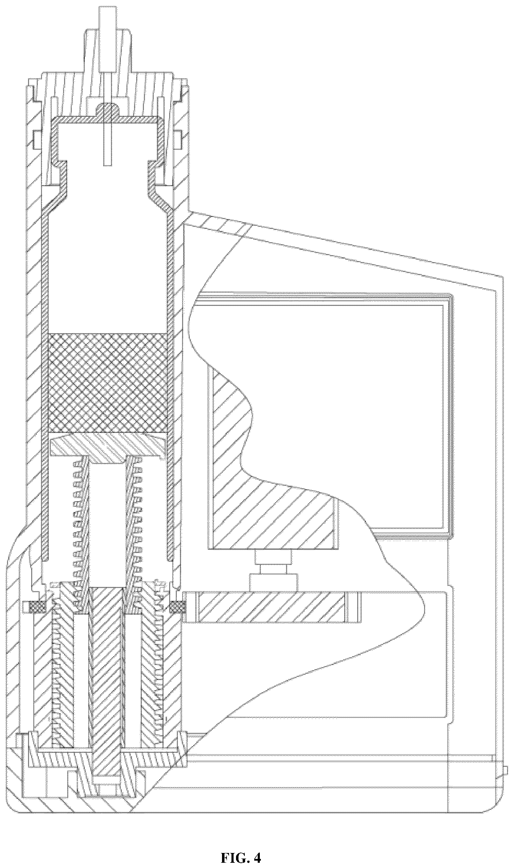

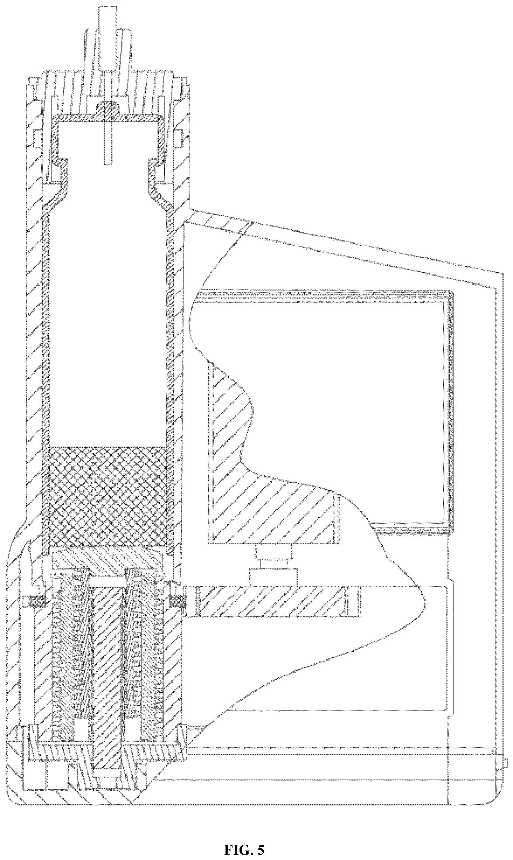

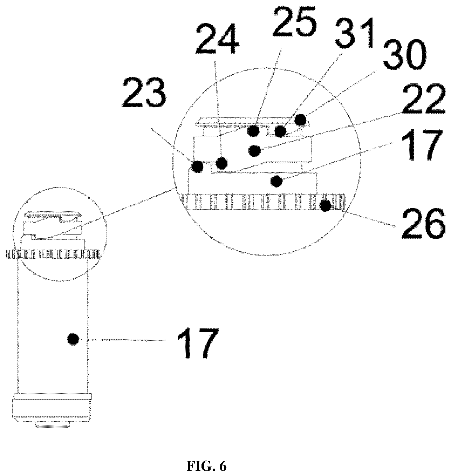

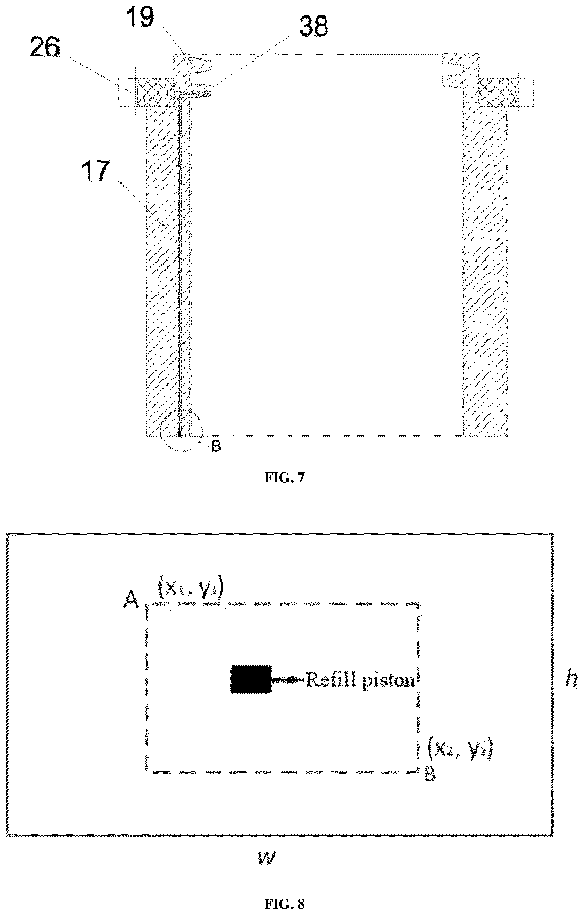

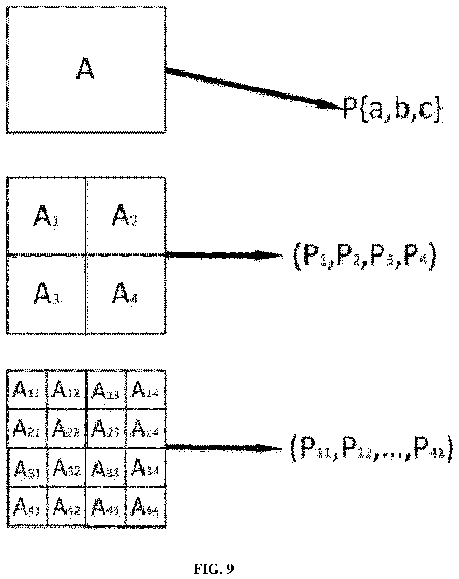

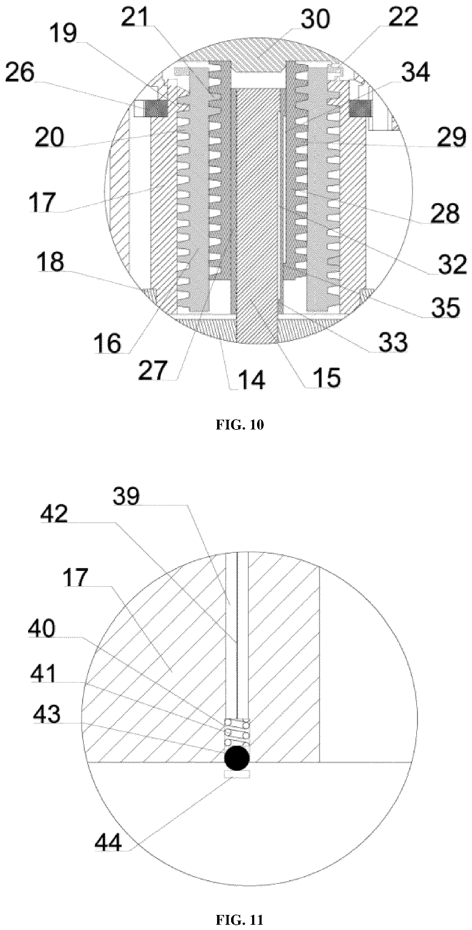

FIGs. 1-11 illustrate an intelligent control method for a refill-type screw insulin injection pump provided in some embodiments of the present disclosure, the refill-type screw insulin injection pump includes a housing 1, a refill injection mechanism, a primary telescopic screw mechanism, a secondary telescopic screw mechanism, a power device, a power supply module and an intelligent control module, where the refill injection mechanism, the primary telescopic screw mechanism and the secondary telescopic screw mechanism are all coaxially and vertically disposed on a left side portion inside the housing, the power device, the power supply module and the intelligent control module are all disposed on a right side portion inside the housing 1, the power device is in transmission connection with the primary telescopic screw mechanism, the power device drives the primary telescopic screw mechanism to telescope, the secondary telescopic screw mechanism is installed inside the primary telescopic screw mechanism and is in transmission connection with the primary telescopic screw mechanism, the primary telescopic screw mechanism drives the secondary telescopic screw mechanism to telescope, the refill injection mechanism is disposed on an upper portion of the secondary telescopic screw mechanism and is connected to an telescopic end of the secondary telescopic screw mechanism, a key operation module 2 is disposed on the housing 1, a sensor assembly is disposed on the primary telescopic screw mechanism, the power supply module supplies power to the power device and the intelligent control module, and the intelligent control module is in signal connection with the power device, the key operation module 2 and the sensor assembly.

A bottom of the housing 1 is open and fixedly connected to a bottom cover 3, an injection cartridge 4 similar to a pen holder structure is integrally formed on a left side of an interior of the housing 1 in a vertical direction, an upper end of the injection cartridge 4 penetrates upwards through a top of the housing 1, a lower end of the injection cartridge 4 extends downwards to a lower left portion of the housing 1, an inner electrical cavity 5 is formed inside a lower right portion of the housing 1, the power supply module and the intelligent control module are disposed inside the inner electrical cavity 5, and the power device is disposed inside the upper right portion of the housing 1.

The refill injection mechanism includes a refill 6 and a refill piston 7, the refill 6 is coaxially and fixedly embedded inside the injection cartridge 4, an outer circle of the refill 6 is in close contact with an inner circle of the injection cartridge 4, a lower end of the refill 6 is open, the lower end of the refill 6 is located inside the injection cartridge 4 and is close to the lower end of the injection cartridge 4, a T-shaped hollow cylindrical head 8 with a large top but a small bottom is integrally formed on an upper top of the refill 6, a maximum circumferential diameter of the T-shaped hollow cylindrical head 8 is smaller than a diameter of the refill 6, a lower end of the T-shaped hollow cylindrical head 8 is connected to the upper top of the refill 6 through a truncated cone tube 9 with a narrow top but a wide bottom, an upper end of the injection cartridge 4 is coaxially and threadedly connected with a needle cap 10, the T-shaped hollow cylindrical head 8 is embedded inside the needle cap 10, a cylindrical protrusion 11 is coaxially and integrally formed in a center of a top of the needle cap 10, a first central hole is vertically formed in a center of the cylindrical protrusion 11, an injection hose 12 is coaxially and fixedly embedded inside the first central hole, an upper end of the injection hose 12 extends out from an upper end of the cylindrical protrusion 11 and is connected to an injection needle, a second central hole coaxial with the first central hole is formed in a center of the needle cap 10, an inner diameter of the second central hole is smaller than an inner diameter of the first central hole, an upper end of the second central hole is connected to a lower end of the first central hole, a barrel 13 is coaxially and fixedly embedded inside the second central hole, the barrel 13 coaxially penetrates through the T-shaped hollow cylindrical head 8 and extends into the T-shaped hollow cylindrical head 8, a lower end of the barrel 13 is communicated with an interior of the refill 6, and the refill piston 7 is coaxially sealed and slidably disposed inside the refill 6.

The primary telescopic screw mechanism includes a fixing base 14, a primary fixing rod 15, a primary tubular screw 16 and a driving cylinder 17, a circular convex edge 18 is integrally formed on an outer edge of an upper surface of the fixing base 14, a third central hole is formed in a center of the fixing base 14, the primary fixing rod 15 is coaxially disposed below the injection cartridge 4, an upper end of the primary fixing rod 15 is located in a center of the lower end of the injection cartridge 4, a lower end of the primary fixing rod 15 is fixedly connected to the third central hole, the primary tubular screw 16 is coaxially sleeved outside the primary fixing rod 15, the driving cylinder 17 is coaxially sleeved outside the primary tubular screw 16 and is disposed between the injection cartridge 4 and the fixing base 14, an upper end of the driving cylinder 17 is rotationally connected to the lower end of the injection cartridge 4, a lower end of the driving cylinder 17 is rotationally connected to the circular convex edge 18, an inner diameter of the driving cylinder 17 is smaller than an inner diameter of the refill 6, first internal thread 19 is formed on an inner circle of the upper end of the driving cylinder 17, first external thread 20 in threaded fit with the first internal thread 19 is axially formed in an outer ring of the primary tubular screw 16, second internal thread 21 is formed on an inner ring of an upper end portion of the primary tubular screw 16, a lower end of the primary tubular screw 16 is flush with the lower end of the driving cylinder 17 and is higher than the upper surface of the fixing base 14, an upper end of the primary tubular screw 16 is higher than the upper end of the driving cylinder 17, a limiting ring 22 located above the upper end of the driving cylinder 17 is fixedly sleeved on an outer circle of the upper end portion of the primary tubular screw 16, a first wedge-shaped clip 23 protruding upwards is disposed on an upper end surface of the driving cylinder 17, and a second wedge-shaped clip 24 protruding downwards is disposed on a lower end surface of the limiting ring 22; and when the primary tubular screw 16 is retracted downwards inside the driving cylinder 17 and moves downwards to a lower limit position, the first wedge-shaped clip 23 is engaged with the second wedge-shaped clip 24, a third wedge-shaped clip 25 protruding downwards is disposed on an upper end surface of the limiting ring 22, and a driving gear 26 is fixedly sleeved on an outer circumference of the upper end of the driving cylinder 17;

the secondary telescopic screw mechanism includes a secondary fixing sleeve 27 and a secondary tubular screw 28, the secondary fixing sleeve 27 is coaxially and slidably sleeved on the primary fixing rod 15, the secondary tubular screw 28 is coaxially and slidably sleeved on the secondary fixing sleeve 27 and is located inside the primary tubular screw 16, second external thread 29 in threaded fit with the second internal thread 21 is axially formed in an outer ring of the secondary tubular screw 28, an upper end of the secondary fixing sleeve 27 is flush with the upper end of the primary fixing rod 15, a lower end of the secondary fixing sleeve 27 is flush with the lower end of the primary tubular screw 16, an upper end of the secondary tubular screw 28 is higher than the upper end of the primary tubular screw 16 and is fixedly connected to a limiting screw plunger 30 located above the limiting ring 22,

a diameter of the limiting screw plunger 30 is smaller than the inner diameter of the refill 6, a top of the limiting screw plunger 30 extends into the refill 6 and is fixedly connected to a bottom of the refill piston 7, and a fourth wedge-shaped clip 31 protruding downwards is disposed on an outer edge of a bottom of the limiting screw plunger 30; and when the secondary tubular screw 28 is retracted downwards inside the primary tubular screw 16 and moves downwards to a lower limit position, the fourth wedge-shaped clip 31 is engaged with the third wedge-shaped clip 25, a lower end of the secondary tubular screw 28 is higher than the lower end of the secondary fixing sleeve 27, a first limiting slot 32 which is open on lower and outer sides is formed on an limiting block of the primary fixing rod 15 in an axial direction, a first limiting block 33 correspondingly and slidably clamped inside the first limiting slot 32 is integrally formed on an inner wall of a lower end portion of the secondary fixing sleeve 27, a second limiting slot 34 which is open on lower and outer sides is formed an outer wall of the secondary fixing sleeve 27 in an axial direction, and a second limiting block 35 correspondingly and slidably clamped inside the second limiting slot 34 is integrally formed on an inner wall of a lower end portion of the secondary tubular screw 28; and

the first internal thread 19, the first external thread 20, the second internal thread 21 and the second external thread 29 are trapezoidal threads with the same thread pitch.

The power device includes a power motor 36, the power motor 36 fixedly mounted on an upper right portion of the housing 1, an output shaft of the power motor 36 is vertically disposed on a lower side of the power motor 36, a motor gear 37 is coaxially and fixedly mounted on a lower end of the output shaft of the power motor 36, the motor gear 37 is located on a right side of the driving gear 26 and is in meshing transmission connection with the driving gear 26, the power module is electrically connected to the power motor 36, and the intelligent control module is in signal connection with the power motor 36.

The sensor assembly includes a position sensor 38, the position sensor 38 is disposed on a lower side face of a trapezoidal thread at a lower end of the first internal thread 19, a wire harness channel 39 is axially formed on a cylinder body of the driving cylinder 17 corresponding to the position sensor 38, a mounting hole 40 with a diameter greater than that of the wire harness channel 39 is coaxially formed on a lower end of the wire harness channel 39, a lower port of the mounting hole 40 is formed on a lower end surface of the driving cylinder 17, an elastic conductor 41 is fixedly disposed inside an upper portion of the mounting hole 40, one first signal wire 42 runs through the wire harness channel 39, an upper end of the first signal wire 42 is connected to the position sensor 38, a lower end of the first signal wire 42 is connected to the elastic conductor 41, a lower end of the elastic conductor 41 is fixedly connected to a conductive rolling ball 43 located at a lower portion inside the mounting hole 40, a lower side portion of the conductive rolling ball 43 protrudes downwards to form a lower port of the mounting hole 40, a conductive sheet 44 is disposed on the upper surface of the fixing base 14, the conductive sheet 44 is in rolling contact with the conductive rolling ball 43 in an up-down correspondence manner, and the conductive sheet 44 is in signal connection with the intelligent control module through a second signal wire passing through a lower surface of the fixing base 14 and is introduced into the inner electrical cavity 5.

The refill 6 is made of transparent material, a camera with built-in light embedded in an inner wall of the injection cartridge 4 is disposed in a middle position of an outer wall of the refill 6, the power module is electrically connected to the camera, and the intelligent control module is in signal connection with the camera.

The intelligent control module is a microcomputer with an embedded processor, and the intelligent control module is further provided with a built-in soft limit of the secondary telescopic screw mechanism, where soft limit parameters thereof are determined according to geometric parameters, that is, thread pitches, total lengths and working lengths, of the primary tubular screw 16 and the secondary tubular screw 28, therefore, the intelligent control module immediately controls the power motor 36 to stop rotating when the second limiting block on the inner wall of the lower end portion of the secondary tubular screw 28 slides upwards to an upper end of the second limiting slot and is retained, and the secondary tubular screw 28 retracts downwards inside the primary tubular screw 16 and moves downwards to the lower limit position.

The intelligent control method specifically includes the following steps:

step (I) start the refill-type screw insulin injection pump and operate the key operation module 2, control the power device to work through the intelligent control module, drive the primary telescopic screw mechanism and the secondary telescopic screw mechanism to telescope by the power device, and fill prefabricated liquid insulin medicine into the refill injection mechanism;

step (II) operate the key operation module 2, control the power device to work through the intelligent control module, and drive the primary telescopic screw mechanism to extend first through the power device; when the primary telescopic screw mechanism extends to a limit, the sensor assembly transmits a signal to the intelligent control module, and the intelligent control module controls a rotational speed of the power device to be slowed down to reduce impact force of the primary telescopic screw mechanism, the secondary telescopic screw mechanism then starts to extend until it extends to a maximum limit position, and the intelligent control module controls the power device to stop; and in the extension process of the primary telescopic screw mechanism and the secondary telescopic screw mechanism, the refill injection mechanism performs the injection of liquid insulin medicine; and step (III) refill liquid insulin medicine upon completion of the injection of liquid insulin medicine and prepare for a next injection.

The step (I) is specifically as follows: the intelligent control module is initialized when the power module is turned on, the injection needle is inserted into an insulin injection bottle, the key operation module 2 is subjected to operation, the intelligent control module controls the power motor 36 to rotate reversely, the output shaft of the power motor 36 drives the driving cylinder 17 to rotate reversely through the motor gear 37 and the driving gear 26, the driving cylinder 17 drives the primary tubular screw 16 to retract downwards inside the driving cylinder 17 through the first internal thread 19 and the first external thread 20, the primary tubular screw 16 then drives the secondary fixing sleeve 27 and the secondary tubular screw 28 to move downwards relative to the primary fixing rod 15, and the secondary tubular screw 28 drives the limiting screw plunger 30 and the refill piston 7 to move downwards, such that the refill piston 7 can draw the liquid insulin medicine in the insulin injection bottle into the refill 6 through the injection needle, the injection hose 12 and the barrel 13; when the primary tubular screw 16 retracts downwards inside the driving cylinder 17 and moves downwards to the lower limit position, the second wedge-shaped clip 24 is engaged with the first wedge-shaped clip 23, and the primary tubular screw 16 cannot continue to move downwards, therefore, the driving cylinder 17 drives the primary tubular screw 16 to rotate in an opposite direction through the first wedge-shaped clip 23 and the second wedge-shaped clip 24, the primary tubular screw 16 and the secondary tubular screw 28 rotate relative to each other, the primary tubular screw 16 drives the secondary tubular screw 28 to retracts downwards into the primary tubular screw 16 through the second internal thread 21 and the second external thread 29, the secondary tubular screw 28 continues to drive the limiting screw plunger 30 and the refill piston 7 to move downwards, and the refill piston 7 continues to draw the liquid insulin medicine in the insulin injection bottle into the refill 6 through the injection needle, the injection hose 12 and the barrel 13; and when the secondary tubular screw 28 retracts downwards into the primary tubular screw 16 and moves downwards to the lower limit position, the fourth wedge-shaped clip 31 is engaged with the third wedge-shaped clip 25, the secondary tubular screw rod 28 cannot continue to move downwards, the intelligent control module immediately controls the power motor 36 to stop rotating, the refill 6 is filled with liquid insulin medicine, the filling operation of the liquid insulin medicine is completed, and a new injection needle head is replaced to prepare for the injection.

The step (II) is specifically as follows: the key operation module 2 is subjected to operation, the intelligent control module controls the power motor 36 to rotate forward, the output shaft of the power motor 36 drives the motor gear 37 to rotate, the motor gear 37 is meshed with and drives the driving gear 26, the driving gear 26 then drives the driving cylinder 17 to rotate forward, the driving cylinder 17 and the primary tubular screw 16 rotate relative to each other, the driving cylinder 17 drives the primary tubular screw 16 to extend out from the driving cylinder 17 through the first internal thread 19 and the first external thread 20, the primary tubular screw 16 then drives the secondary fixing sleeve 27 and the secondary tubular screw 28 to move upwards relative to the primary fixing rod 15, the first limiting block 33 on the inner wall of the lower end portion of the secondary fixing sleeve 27 slides upwards in the first limiting slot 32 on the outer side wall of the primary fixing rod 15, the secondary tubular screw 28 pushes the limiting screw plunger 30 and the refill piston 7 to move upwards, such that the refill piston 7 pushes out the liquid insulin medicine in the refill 6 and performs the injection through the barrel 13, the injection hose 12 and the injection needle; since the first internal thread 19, the first external thread 20, the second internal thread 21 and the second external thread 29 are trapezoidal threads with the same thread pitch, one full rotation of the driving cylinder 17 can make the primary tubular screw 16 move upwards by a length of one thread pitch, and the conductive rolling ball 43 is in rolling contact with the conductive sheet 44, in which case, the position sensor 38 transmits a signal to the intelligent control module once, the intelligent control module obtains information about the number of rotations of the driving cylinder 17 by acquiring the signal from the position sensor 38, the intelligent control module obtains a distance that the primary tubular screw 16 moves upwards according to the number of rotations of the driving cylinder 17, meanwhile, the intelligent control module processes images captured by the camera through a pyramid YOLO7 algorithm to identify a position of the refill piston 7, an injected amount of liquid insulin medicine in the refill 6 is then measured, the intelligent control module controls the power motor 36 to change a rotational torque and speed, impact face between the primary tubular screw 16 and the driving cylinder 17 is reduced until the primary tubular screw 16 moves upwards to an upper limit position, that is, the primary tubular screw 16 and the driving cylinder 17 cannot continue to rotate relative to each other; when the first limiting block 33 slides upwards to an upper end of the first limiting slot 32 and is retained, the primary tubular screw 16 is in transmission connection with the driving cylinder 17 to form an entirety, an movement form of the primary tubular screw 16 changes from moving up and down in a vertical direction to be rotated together with the driving cylinder 17, the primary tubular screw 16 and the secondary tubular screw 28 rotate relative to each other, the primary tubular screw 16 drives the secondary tubular screw 28 to extend upwards and out from the primary tubular screw 16 through the second internal thread 21 and the second external thread 29, the secondary tubular screw 28 moves upwards relative to the secondary fixing sleeve 27, the secondary tubular screw 28 continues to push the limiting screw plunger 30 and the refill piston 7 to move upwards, and the refill piston 7 continues to push out the liquid insulin medicine in the refill 6 and is injected through the barrel 13, the injection hose 12 and the injection needle; and when the second limiting block 35 on the inner wall of the lower end of the secondary tubular screw 28 slides upwards to the upper end of the second limiting slot 34 and is retained, the secondary tubular screw 28 moves upward to its upper limit position, the primary tubular screw 16 and the secondary tubular screw 28 cannot continue to rotate relative to each other, the intelligent control module controls the power motor 36 to stop rotating, and the whole injection process of the liquid insulin medicine is completed.

The intelligent control module controls the power motor 36 to change the rotational torque and speed through the following action flow:

A) when the number of signals acquired by the intelligent control module from the position sensor 38 reaches a first set number of times, the number of rotations of the driving cylinder 17 reaches the first set number of rotations, the primary tubular screw 16 moves upward to start to approach its upper limit position state, the intelligent control module measures through the camera that the injected amount of liquid insulin medicine in the refill 6 is greater than one third or start to approach one half, and the intelligent control module controls driving current of the power motor 36 to be increased to 125% of a standard state (50 mA) through the camera, improves the rotational torque of the power motor 36, and reduces the speed of the power motor 36 to 80% of a standard state (200 rpm/Sec) through PWM speed regulation;

B) when the driving cylinder 17 continues to rotate forward, the number of signals acquired by the intelligent control module from the position sensor 38 reaches a second set number of times, the number of rotations of the driving cylinder 17 reaches the second set number of rotations, the primary tubular screw 16 moves upward to further approach its upper limit position state, the intelligent control module measures through the camera that the refill piston 7 further moves upward and that the injected amount of liquid insulin medicine in the refill 6 further approaches one half, and the intelligent control module controls driving current of the power motor 36 to be increased to 150% of the standard state (50 mA) through the camera, improves the rotational torque of the power motor 36, and reduces the speed of the power motor 36 to 60% of the standard state (200 rpm/Sec) through PWM speed regulation;

C) when the driving cylinder 17 continues to rotate forward, the number of signals acquired by the intelligent control module from the position sensor 38 reaches a third set number of times, the number of rotations of the driving cylinder 17 reaches the third set number of rotations, the primary tubular screw 16 moves upward to almost approach its upper limit position state, the intelligent control module measures through the camera that the refill piston 7 further moves upward and that the injected amount of liquid insulin medicine in the refill 6 almost approaches one half, and the intelligent control module controls driving current of the power motor 36 to be increased to 200% of the standard state (50 mA) through the camera, improves the rotational torque of the power motor 36, reduces the speed of the power motor 36 to 20% of the standard state (200 rpm/Sec) through PWM speed regulation, and reduces the impact force of the primary tubular screw 16 and the driving cylinder 17; and

D) when the driving cylinder 17 continues to rotate forward, the number of signals acquired by the intelligent control module from the position sensor 38 reaches a fourth set number of times, the number of rotations of the driving cylinder 17 reaches the fourth set number of rotations, the primary tubular screw 16 moves upward to reach its upper limit position state, the intelligent control module measures through the camera that the refill piston 7 further moves upward and that the injected amount of liquid insulin medicine in the refill 6 further reaches one half, and the intelligent control module controls driving current of the power motor 36 to be increased to 300% of the standard state (50 mA) through the camera, improves the rotational torque of the power motor 36, reduces the speed of the power motor 36 to 5% of the standard state (200 rpm/Sec) through PWM speed regulation, and further reduces the impact force of the primary tubular screw 16 and the driving cylinder 17.

An input image of the pyramid YOLO7 algorithm is the image captured by the camera, a resolution a real-time monitoring screen image of the camera is w × h pixels, where a value of w is 1280 pixels, a value of h is 720 pixels, the pyramid YOLO7 algorithm performs rectangular window cropping on keyframes of the w × h pixels of an original input image, a starting point and an ending point of the rectangular window cropping are respectively denoted as $A(x_1,y_1)$ and $B(x_2,y_2)$, and the two points are obtained by the following method:

(1) historically detected data is analyzed to obtain coordinates of a maximum circumscribed rectangle area of a target object hotspot marked by the pyramid YOLO7 algorithm with a self-adaptive weight over the most recent 7 days, which are respectively denoted as: $S_1\{(x_{11},y_{11}), (x_{21},y_{21})\}$, $S_2\{(x_{12},y_{12}), (x_{22},y_{22})\}$, $S_3\{(x_{13},y_{13}), (x_{23},y_{23})\}...S_n\{(x_{1n},y_{1n}), (x_{2n},y_{2n})\}$;

coordinates of an X direction and a Y direction of the starting point A $(x_1, y_1)$ are expressed as Formulae (1) and (2) below:

$$x_1 = 0.8 * (x'_{11} + x'_{12} + x'_{13} + \cdots + x'_{1[n/2]})/[n/2] \quad (1);$$

$$y_1 = 0.8 * (y'_{11} + y'_{12} + y'_{13} + \cdots + y'_{1[n/2]})/[n/2] \quad (2);$$

where [n/2] is n/2 rounded down, and $x_1$, $y_1$ are both greater than and equal to 1; $x'_{11}, x'_{12}, x'_{13} \ldots \ldots x'_{1[n/2]}$ , and $y'_{11}$ , $y'_{12}, y'_{13} \ldots \ldots y'_{1[n/2]}$ are data series $x_{11}$, $x_{12}$, $x_{13}, \ldots \ldots x_{1n}$ and $y_{11}$, $y_{12}$, $y_{13}$, $\ldots \ldots y_{1n}$ mare arranged in ascending order respectively, and the first 50% of the data series are taken;

coordinates of an X direction and a Y direction of the ending point B $(x_2, y_2)$ are expressed as Formulae (3) and (4) below:

$$x_2 = 0.8 * (x'_{21} + x'_{22} + x'_{23} + \cdots + x'_{2[n/2]})/[n/2] \quad (3);$$

$$y_2 = 0.8 * (y'_{21} + y'_{22} + y'_{23} + \cdots + y'_{2[n/2]})/[n/2] \quad (4);$$

where $x_2$ is less than and equals to 1280, $y_2$ is less than and equals to 720; $x'_{21}, x'_{22}, x'_{23} \ldots x'_{2[n/2]}$ and $y'_{21}, y'_{22}$ , $y'_{23} \ldots y'_{2[n/2]}$ are data series $x_{21}$, $x_{22}$, $x_{23}, \ldots x_{2n}$ and $y_{21}$, $y_{22}$, $y_{23}$, $\ldots y_{2n}$ are arranged in ascending order, and the first 50% of the data series are taken;

(2) when the cumulative number of days of historically data is less than 7 days, coordinates of the starting point A are set as (1, 1), and coordinates of the ending point B are set as (1280, 720).

Specifically, the process of the pyramid YOLO7 algorithm is as follows:

step I. when the refill-type screw insulin injection pump performs the injection of liquid insulin medicine, the intelligent control module acquires images captured by the camera once every 10 ms, and the pyramid YOLO7 algorithm determines whether an image threshold is greater than 5 ml/h; and when the image threshold is greater than 5 ml/h, the next step II will be performed, otherwise, the current step I will be continuously performed;

step II. when a current injected amount is less than half of a capacity of the refill 6, an image of which keyframes captured by the camera at a current moment are subjected to the rectangular window cropping are acquired and marked as Image A; the current moment is taken as a starting point, and images of which keyframes over the previous 500 ms and the previous 2 s are subjected to the rectangular window cropping are acquired, which are marked as Images B and C, respectively, and then the next step III is performed;

step III. first, the pyramid YOLO7 algorithm performs YOLO 7 target object detection on Image A, and a maximum probability that the injection amount is less than or equals to 0, is greater than 0 but less than 1/2 and equals to 1 in the current images is calculated, and is denoted as P{a, b, c};

Image A is divided by two equal parts into four sub-images in length and width directions, and a maximum probability that the injection amount is less than or equals to 0, is greater than 0 but less than 1/2 and equals to 1 in the current sub-images is obtained and is denoted as $P_1$, $P_2$, $P_3$ and $P_4$; and maximum values of $P_1$, $P_2$, $P_3$ and $P_4$ of the four sub-images in each dimension is selected and denoted as $P_5${d, e, f};

Image A is divided by four equal parts into sixteen sub-images in length and width directions, and a maximum probability that the injection amount is less than or equals to 0, is greater than 0 but less than 1/2 and equals to 1 in the current sub-images is obtained and is denoted as $P_{11} \ldots P_{14} \ldots P_{24} \ldots P_{44}$; and maximum values of $P_{11} \ldots P_{14} \ldots P_{24} \ldots P_{44}$ of the sixteen sub-images in each dimension is selected and denoted as $P_{55}${g, h, i};

a target detection probability $P_A$ of Image A is obtained by identifying through the pyramid YOLO7 algorithm in the three layers above:

$$P_A = \left\{ \frac{1}{3} \times (w_1 \times a + w_2 \times d + w_3 \times g), \frac{1}{3} \times (w_4 \times b + w_5 \times e + w_6 \times h), \frac{1}{3} \times (w_7 \times c + w_8 \times f + w_9 \times i) \right\}$$

(5);

where $w_1......w_9$ are weights and are obtained by the following formulae:

$$w_1 = \frac{a}{a+b+c}, \quad w_2 = \frac{d}{d+e+f}, \quad w_3 = \frac{g}{g+h+i} \quad (6);$$

$$w_4 = \frac{b}{a+b+c}, \quad w_5 = \frac{e}{d+e+f}, \quad w_6 = \frac{h}{g+h+i} \quad (7);$$

and

$$w_7 = \frac{c}{a+b+c}, \quad w_8 = \frac{f}{d+e+f}, \quad w_9 = \frac{i}{g+h+i} \quad (8).$$

step IV. Images B and C are processed with reference to the pyramid YOLO7 algorithm with the self-adaptive weight for Image A, and final target detection probabilities of Images B and C are obtained and denoted as $P_B$ and Pc;
step V. the maximum dimension values of the final target detection probabilities $P_A$, $P_B$ and $P_C$ corresponding to the three images A, B and C are identified, and final detection results of an injection speed and an injection amount at the current moment are obtained according to a maximum value determination principle, that is, less than or equaling to 0, greater than 0 but less than 1/2 and equaling to 1, for example, when calculation results are $P_A$={1, 0, 0}, $P_B$={0.56, 0.16, 0}, Pc={0.46, 0.09, 0}, a maximum value of $P_A$, $P_B$ and $P_C$ appears in the first dimension, therefore, it is determined that the current injection amount is less than or equals to 0 according to the maximum value determination principle; and
step VI. after the detection is completed, a next round of detection is started again, and the step I is performed for processing.

In the present disclosure, the first internal thread 19 is formed on the inner circle of the upper end of the driving cylinder 17, the first external thread 20 in threaded fit with the first internal thread 19 is axially formed in the outer ring of the primary tubular screw 16, the second internal thread 21 is formed on the inner ring of the upper end portion of the primary tubular screw 16, the second external thread 29 in threaded fit with the second internal thread 21 is axially formed in the outer ring of the secondary tubular screw 28, and the first internal thread 19, the first external thread 20, the second internal thread 21 and the second external thread 29 are trapezoidal threads with the same thread pitch, therefore, one full rotation of the driving cylinder 17 can make the primary tubular screw 16 or the secondary tubular screw 28 extend or retract by a length of one thread pitch, such that the injection amount of liquid insulin medicine can be accurately controlled, high-precision medicine delivery can be achieved, and an overall size of the refill-type screw insulin injection pump is significantly reduced, therefore, during the whole injection process of the liquid insulin medicine, the intelligent control module can calculate the number of rotations of the driving cylinder 17 by acquiring the signal from the position sensor 38, the intelligent control module obtains a distance that the primary tubular screw 16 moves upwards according to the number of rotations of the driving cylinder 17, and meanwhile, the intelligent control module processes images captured by the camera through a pyramid YOLO7 algorithm to identify a position of the refill piston 7, an injected amount of liquid insulin medicine in the refill 6 is then measured, the intelligent control module controls the power motor 36 to change a rotational torque and speed, and impact face between the primary tubular screw 16 and the driving cylinder 17 is reduced; and the intelligent control module is further provided with a built-in soft limit of the secondary telescopic screw mechanism, where soft limit parameters thereof are determined according to geometric parameters, that is, thread pitches, total lengths and working lengths, of the primary tubular screw 16 and the secondary tubular screw 28, therefore, the intelligent control module immediately controls the power motor 36 to stop rotating when the second limiting block on the inner wall of the lower end portion of the secondary tubular screw 28 slides upwards to an upper end of the second limiting slot and is retained, and the secondary tubular screw 28 retracts downwards inside the primary tubular screw 16 and moves downwards to the lower limit position, such that the secondary tubular screw 28 is prevented from being tensioned or the secondary tubular screw 28 enters the primary tubular screw 16 and becomes too tight and is locked, and meanwhile, the power motor 36 is prevented from being damaged due to too large torque of the output shaft caused by overloading of the power motor, and the effect of protecting the power motor 36, the primary tubular screw 16 and the secondary tubular screw 28 are achieved.
When the present disclosure is filled with the liquid insulin medicine, the power motor 36 drives the driving cylinder 17 to rotate reversely through the motor gear 37 and the driving gear 26, such that the primary tubular screw 16 is driven to retract downwards inside the driving cylinder 17; since a limiting ring 22 located above the upper end of the driving cylinder 17 is fixedly sleeved on an outer circle of the upper end portion of the primary tubular screw 16, a first wedge-shaped clip 23 protruding upwards is disposed on an upper end surface of the driving cylinder 17, a second wedge-shaped clip 24 protruding downwards is disposed on a lower end surface of the limiting ring 22, a third wedge-shaped clip 25 protruding downwards is disposed on an upper end surface of the limiting ring 22, and a fourth wedge-shaped clip 31 protruding downwards is disposed on an outer edge of a bottom of the limiting screw plunger 30; when the primary tubular screw 16 retracts downwards inside the driving cylinder 17 and moves downwards to the lower limit position, the second wedge-shaped clip 24 is engaged with the first wedge-shaped clip 23, in which case, the primary tubular screw 16 rotates reversely together with the driving cylinder 17 to prevent the primary tubular screw 16 from entering the driving cylinder 17 deeply or tightly, resulting in being locked; the primary tubular screw 16 and the secondary tubular screw 28 rotate relative to each other, the primary tubular screw 16 drives the secondary tubular screw 28 to retracts downwards into the primary tubular screw 16 through the second internal thread 21 and the second external thread 29, and similarly when the secondary tubular screw 28 retracts downwards inside the primary tubular screw 16 and moves downwards to the lower limit position, the fourth wedge-shaped clip 31 is engaged with the third wedge-shaped clip 25, the secondary tubular screw rod 28 cannot continue to move downwards and retract into the primary tubular screw 16, in which case, the second wedge-shaped clip 24 is engaged with the first wedge-shaped clip 23, and the fourth wedge-shaped clip 31 is engaged with the third wedge-shaped clip 25, such that an anti-locking effect can be achieved in the structure.

In this embodiment, the power supply module, the intelligent control module, the injection needle and the camera are not shown in the accompanying drawings, and the power supply module, the intelligent control module, the key operation module 2, the power motor 36, the injection needle and the camera are all conventional techniques, and the specific structure and working principle thereof will not be described in detail herein; and the control and calculation methods built into the intelligent control module of the present disclosure are conventional technologies and do not involve any new computer programs.

The above embodiments are merely intended for describing rather than limiting the technical solutions of the present disclosure. Although the present disclosure is described in detail with reference to the above embodiments, those ordinarily skilled in the art should understand that they may still make modifications or equivalent substitutions to the present disclosure without departing from any modifications or partial substitutions of the present disclosure, which shall fall within the scope of the claims of the present disclosure

**Claims**

1.  An intelligent control method for a refill-type screw insulin injection pump, **characterized by** comprising a housing, a refill injection mechanism, a primary telescopic screw mechanism, a secondary telescopic screw mechanism, a power device, a power supply module and an intelligent control module;

    the refill injection mechanism, the primary telescopic screw mechanism and the secondary telescopic screw mechanism are all coaxially and vertically disposed on a left side portion inside the housing, the power device, the power supply module and the intelligent control module are all disposed on a right side portion inside the housing, the power device is in transmission connection with the primary telescopic screw mechanism, the power device drives the primary telescopic screw mechanism to telescope, the secondary telescopic screw mechanism is installed inside the primary telescopic screw mechanism and is in transmission connection with the primary telescopic screw mechanism, the primary telescopic screw mechanism drives the secondary telescopic screw mechanism to telescope, the refill injection mechanism is disposed on an upper portion of the secondary telescopic screw mechanism and is connected to an telescopic end of the secondary telescopic screw mechanism, a key operation module is disposed on the housing, a sensor assembly is disposed on the primary telescopic screw mechanism, the power supply module supplies power to the power device and the intelligent control module, and the intelligent control module is in signal connection with the power device, the key operation module and the sensor assembly, respectively;
    a bottom of the housing is open and fixedly connected to a bottom cover, an injection cartridge similar to a pen holder structure is integrally formed on a left side of an interior of the housing in a vertical direction, an upper end of the injection cartridge penetrates upwards through a top of the housing upwards, a lower end of the injection cartridge extends downwards to a lower left portion of the housing, an inner electrical cavity is formed inside a lower right portion of the housing, the power supply module and the intelligent control module are disposed inside the inner electrical cavity, and the power device is disposed inside the upper right portion of the housing; **characterized in that** the intelligent control method comprises the following steps:

    step (I). starting the refill-type screw insulin injection pump, operating the key operation module to enable the intelligent control module to control the power device to work, and to enable the power device to drive the primary telescopic screw mechanism and the secondary telescopic screw mechanism to telescope, and allowing prefabricated liquid insulin medicine to be filled into the refill injection mechanism;

step (II). controlling the power device to work through the intelligent control module, and driving the primary telescopic screw mechanism to extend first through the power device; when the primary telescopic screw mechanism extends to a limit, the sensor assembly transmits a signal to the intelligent control module, and the intelligent control module controls a rotational speed of the power device to be slowed down to reduce impact force of the primary telescopic screw mechanism, the secondary telescopic screw mechanism then starts to extend until it extends to a maximum limit position, and the intelligent control module controls the power device to stop; and in the extension process of the primary telescopic screw mechanism and the secondary telescopic screw mechanism, the refill injection mechanism performs the injection of liquid insulin medicine; and

step (III). repeating the step (I) upon completion of the injection of liquid insulin medicine, and refilling liquid insulin medicine to prepare for a next injection.

2. The intelligent control method for a refill-type screw insulin injection pump according to claim 1, **characterized in that** the refill injection mechanism comprises a refill and a refill piston, the refill is coaxially and fixedly embedded inside the injection cartridge, an outer circle of the refill is in close contact with an inner circle of the injection cartridge, a lower end of the refill is open, the lower end of the refill is located inside the injection cartridge and is close to the lower end of the injection cartridge, a T-shaped hollow cylindrical head with a large top but a small bottom is integrally formed on an upper top of the refill, a maximum circumferential diameter of the T-shaped hollow cylindrical head is smaller than a diameter of the refill, a lower end of the T-shaped hollow cylindrical head is connected to the upper top of the refill through a truncated cone tube with a narrow top but a wide bottom; and the upper end of the injection cartridge is coaxially and threadedly connected with a needle cap, the T-shaped hollow cylindrical head is embedded inside the needle cap, a cylindrical protrusion is coaxially and integrally formed in a center of a top of the needle cap, a first central hole is vertically formed in a center of the cylindrical protrusion, an injection hose is coaxially and fixedly embedded inside the first central hole, an upper end of the injection hose extends out from an upper end of the cylindrical protrusion and is connected to an injection needle, a second central hole coaxial with the first central hole is formed in a center of the needle cap, an inner diameter of the second central hole is smaller than an inner diameter of the first central hole, an upper end of the second central hole is connected to a lower end of the first central hole, a barrel is coaxially and fixedly embedded inside the second central hole, the barrel coaxially penetrates through the T-shaped hollow cylindrical head and extends into the T-shaped hollow cylindrical head, a lower end of the barrel is communicated with an interior of the refill, and the refill piston is coaxially sealed and slidably disposed inside the refill.

3. The intelligent control method for a refill-type screw insulin injection pump according to claim 2, **characterized in that** the primary telescopic screw mechanism comprises a primary fixing rod, a primary tubular screw, a driving cylinder and a fixing base, a circular convex edge is integrally formed on an outer edge of an upper surface of the fixing base, a third central hole is formed in a center of the fixing base, the primary fixing rod is coaxially disposed below the injection cartridge, an upper end of the primary fixing rod is located in a center of the lower end of the injection cartridge, a lower end of the primary fixing rod is fixedly connected to the third central hole, the primary tubular screw is coaxially sleeved outside the primary fixing rod, the driving cylinder is coaxially sleeved outside the primary tubular screw and is disposed between the injection cartridge and the fixing base, an upper end of the driving cylinder is rotationally connected to the lower end of the injection cartridge, a lower end of the driving cylinder is rotationally connected to the circular convex edge, an inner diameter of the driving cylinder is smaller than an inner diameter of the refill, first internal thread is formed on an inner circle of the upper end of the driving cylinder, first external thread in threaded fit with the first internal thread is axially formed in an outer ring of the primary tubular screw, second internal thread is formed on an inner ring of an upper end portion of the primary tubular screw, a lower end of the primary tubular screw is flush with the lower end of the driving cylinder and is higher than the upper surface of the fixing base, an upper end of the primary tubular screw is higher than the upper end of the driving cylinder, a limiting ring located above the upper end of the driving cylinder is fixedly sleeved on an outer circle of the upper end portion of the primary tubular screw, a first wedge-shaped clip protruding upwards is disposed on an upper end surface of the driving cylinder, and a second wedge-shaped clip protruding downwards is disposed on a lower end surface of the limiting ring; and when the primary tubular screw is retracted downwards inside the driving cylinder and moves downwards to a lower limit position, the first wedge-shaped clip is engaged with the second wedge-shaped clip, a third wedge-shaped clip protruding downwards is disposed on an upper end surface of the limiting ring, and a driving gear is fixedly sleeved on an outer circumference of the upper end of the driving cylinder;

the secondary telescopic screw mechanism comprises a secondary fixing sleeve and a secondary tubular screw, the secondary fixing sleeve is coaxially and slidably sleeved on the primary fixing rod, the secondary tubular screw is coaxially and slidably sleeved on the secondary fixing sleeve and is located inside the primary tubular screw, second external thread in threaded fit with the second internal thread is axially formed in an outer ring of the secondary tubular screw, an upper end of the secondary fixing sleeve is flush with the upper end of the primary fixing rod, a lower end of the secondary fixing sleeve is flush with the lower end of the primary tubular screw, an upper end of the secondary tubular screw is higher than the upper end of the primary tubular screw and is fixedly connected to a limiting screw plunger located above the limiting ring, a diameter of the limiting screw plunger is smaller than the inner diameter of the refill, a top of the limiting screw plunger extends into the refill and is fixedly connected to a bottom of the refill piston, and a fourth wedge-shaped clip protruding downwards is disposed on an outer edge of a bottom of the limiting screw plunger; and when the secondary tubular screw is retracted downwards inside the primary tubular screw and moves downwards to a lower limit position, the fourth wedge-shaped clip is engaged with the third wedge-shaped clip, a lower end of the secondary tubular screw is higher than the lower end of the secondary fixing sleeve, a first limiting slot which is open on lower and outer sides is formed on an limiting block of the primary fixing rod in an axial direction, a first limiting block correspondingly and slidably clamped inside the first limiting slot is integrally formed on an inner wall of a lower end portion of the secondary fixing sleeve, a second limiting slot which is open on lower and outer sides is formed an outer wall of the secondary fixing sleeve in an axial direction, and a second limiting block correspondingly and slidably clamped inside the second limiting slot is integrally formed on an inner wall of a lower end portion of the secondary tubular screw; and

the first internal thread, the first external thread, the second internal thread and the second external thread are trapezoidal threads with the same thread pitch.

4. The intelligent control method for a refill-type screw insulin injection pump according to claim 3, **characterized in that**

the power device comprises a power motor, the power motor fixedly mounted on an upper right portion of the housing, an output shaft of the power motor is vertically disposed on a lower side of the power motor, a motor gear is coaxially and fixedly mounted on a lower end of the output shaft of the power motor, the motor gear is located on a right side of the driving gear and is in meshing transmission connection with the driving gear, the power module is electrically connected to the power motor, and the intelligent control module is in signal connection with the power motor;

the sensor assembly comprises a position sensor, the position sensor is disposed on a lower side face of a trapezoidal thread at a lower end of the first internal thread, a wire harness channel is axially formed on a cylinder body of the driving cylinder corresponding to the position sensor, a mounting hole with a diameter greater than that of the wire harness channel is coaxially formed on a lower end of the wire harness channel, a lower port of the mounting hole is formed on a lower end surface of the driving cylinder, an elastic conductor is fixedly disposed inside an upper portion of the mounting hole, one first signal wire runs through the wire harness channel, an upper end of the first signal wire is connected to the position sensor, a lower end of the first signal wire is connected to the elastic conductor, a lower end of the elastic conductor is fixedly connected to a conductive rolling ball located at a lower portion inside the mounting hole, a lower side portion of the conductive rolling ball protrudes downwards to form a lower port of the mounting hole, a conductive sheet is disposed on the upper surface of the fixing base, the conductive sheet is in rolling contact with the conductive rolling ball in an up-down correspondence manner, and the conductive sheet is in signal connection with the intelligent control module through a second signal wire passing through a lower surface of the fixing base and is introduced into the inner electrical cavity;

the refill is made of transparent material, a camera with built-in light embedded in an inner wall of the injection cartridge is disposed in a middle position of an outer wall of the refill, the power module is electrically connected to the camera, and the intelligent control module is in signal connection with the camera; and

the intelligent control module is a microcomputer with an embedded processor, and the intelligent control module is further provided with a built-in soft limit of the secondary telescopic screw mechanism, wherein soft limit parameters thereof are determined according to geometric parameters, that is, thread pitches, total lengths and working lengths, of the primary tubular screw and the secondary tubular screw, the intelligent control module immediately controls the power motor to stop rotating when the second limiting block on the inner wall of the lower end portion of the secondary tubular screw slides upwards to an upper end of the second limiting slot and is retained, and the secondary tubular screw retracts downwards inside the primary tubular screw and moves downwards to the lower limit position.

5. The intelligent control method for a refill-type screw insulin injection pump according to claim 4, **characterized in that** the step (I) is specifically as follows:

the intelligent control module is initialized when the power module is turned on, the injection needle is inserted into an insulin injection bottle, the key operation module is subjected to operation, the intelligent control module controls the power motor to rotate reversely, the output shaft of the power motor drives the driving cylinder to rotate reversely through the motor gear and the driving gear, the driving cylinder drives the primary tubular screw to retract downwards inside the driving cylinder through the first internal thread and the first external thread, the primary tubular screw then drives the secondary fixing sleeve and the secondary tubular screw to move downwards relative to the primary fixing rod, and the secondary tubular screw drives the limiting screw plunger and the refill piston to move downwards, such that the refill piston can draw the liquid insulin medicine in the insulin injection bottle into the refill through the injection needle, the injection hose and the barrel;

when the primary tubular screw retracts downwards inside the driving cylinder and moves downwards to the lower limit position, the second wedge-shaped clip is engaged with the first wedge-shaped clip, and the primary tubular screw cannot continue to move downwards, therefore, the driving cylinder drives the primary tubular screw to rotate in an opposite direction through the first wedge-shaped clip and the second wedge-shaped clip, the primary tubular screw and the secondary tubular screw rotate relative to each other, the primary tubular screw drives the secondary tubular screw to retracts downwards into the primary tubular screw through the second internal thread and the second external thread, the secondary tubular screw continues to drive the limiting screw plunger and the refill piston to move downwards, and the refill piston continues to draw the liquid insulin medicine in the insulin injection bottle into the refill through the injection needle, the injection hose and the barrel; and

when the secondary tubular screw retracts downwards into the primary tubular screw and moves downwards to the lower limit position, the fourth wedge-shaped clip is engaged with the third wedge-shaped clip, the refill is filled with the liquid insulin medicine, the filling operation of the liquid insulin medicine is completed, and a new injection needle head is replaced to prepare for the injection.

6. The intelligent control method for a refill-type screw insulin injection pump according to claim 5, **characterized in that** the step (II) is specifically as follows:

the key operation module is subjected to operation, the intelligent control module controls the power motor to rotate forward, the output shaft of the power motor drives the motor gear to rotate, the motor gear is meshed with and drives the driving gear, the driving gear then drives the driving cylinder to rotate forward, the driving cylinder and

the primary tubular screw rotate relative to each other, the driving cylinder drives the primary tubular screw to extend out from the driving cylinder through the first internal thread and the first external thread, the primary tubular screw then drives the secondary fixing sleeve and the secondary tubular screw to move upwards relative to the primary fixing rod, the first limiting block on the inner wall of the lower end portion of the secondary fixing sleeve slides upwards in the first limiting slot on the outer side wall of the primary fixing rod, and the secondary tubular screw pushes the limiting screw plunger and the refill piston to move upwards, such that the refill piston pushes out the liquid insulin medicine in the refill and performs the injection through the barrel, the injection hose and the injection needle; and

the intelligent control module acquires the signal from the position sensor, obtains information about the number of rotations of the driving cylinder, and obtains a distance that the primary tubular screw moves upwards according to the number of rotations of the driving cylinder; and meanwhile, the intelligent control module processes the collected images through a pyramid YOLO7 algorithm to identify a position of the refill piston, an injected amount of liquid insulin medicine in the refill is then measured, the intelligent control module controls the power motor to change a rotational torque and speed, impact face between the primary tubular screw and the driving cylinder is reduced until the primary tubular screw moves upwards to an upper limit position; when the first limiting block slides upwards to an upper end of the first limiting slot and is retained, the primary tubular screw is in transmission connection with the driving cylinder to form an entirety, an movement form of the primary tubular screw changes from moving up and down in a vertical direction to be rotated together with the driving cylinder, the primary tubular screw and the secondary tubular screw rotate relative to each other, the primary tubular screw drives the secondary tubular screw to extend upwards and out from the primary tubular screw through the second internal thread and the second external thread, the secondary tubular screw moves upwards relative to the secondary fixing sleeve, the secondary tubular screw continues to push the limiting screw plunger and the refill piston to move upwards, and the refill piston continues to push out the liquid insulin medicine in the refill and is injected through the barrel, the injection hose and the injection needle; and when the second limiting block on the inner wall of the lower end of the secondary tubular screw slides upwards to the upper end of the second limiting slot and is retained, the secondary tubular screw moves upward to its upper limit position, the intelligent control module controls the power motor to stop rotating, and the whole injection process of the liquid insulin medicine is completed.

7. The intelligent control method for a refill-type screw insulin injection pump according to claim 6, **characterized in that**

the intelligent control module controls the power motor to change the rotational torque and speed through the following methods:

when the driving cylinder rotates forward to reach a first set number of times, the primary tubular screw moves upward to start to approach its upper limit position state, the intelligent control module measures through the camera that the injected amount of liquid insulin medicine in the refill is greater than one third or start to approach one half, and the intelligent control module controls driving current of the power motor to be increased to 125% of a standard state through the camera, improves the rotational torque of the power motor, and reduces the speed of the power motor to 80% of a standard state through PWM speed regulation;

when the driving cylinder rotates forward to reach a second set number of times, the primary tubular screw moves upward to continue to approach its upper limit position state, the intelligent control module recognizes through the camera that the refill piston moves further upwards, and measures that the injected amount of liquid insulin medicine in the refill further approaches one half, and the intelligent control module controls driving current of the power motor to be increased to 150% of a standard state through the camera, improves the rotational torque of the power motor, and reduces the speed of the power motor to 60% of a standard state through PWM speed regulation;

when the driving cylinder rotates forward to reach a third set number of times, the primary tubular screw moves upward to continue to approach its upper limit position state, the intelligent control module recognizes through the camera that the refill piston moves further upwards, and measures that the injected amount of liquid insulin medicine in the refill approaches one half, and the intelligent control module controls driving current of the power motor to be increased to 200% of a standard state through the camera, improves the rotational torque of the power motor, reduces the speed of the power motor to 20% of a standard state through PWM speed regulation, and further reduces the impact force of the primary tubular screw and the driving cylinder; and

when the driving cylinder rotates forward to reach a fourth set number of times, the primary tubular screw moves upward to reach its upper limit position state, the intelligent control module recognizes through the camera that the refill piston moves further upwards, and measures that the injected amount of liquid insulin medicine in the refill further reaches one half, and the intelligent control module controls driving current of the power motor to be increased to 300% of a standard state through the camera, improves the rotational torque of the power motor, and reduces the speed of the power motor to 5% of a standard state through PWM speed regulation.

8. The intelligent control method for a refill-type screw insulin injection pump according to claim 6, **characterized in that** the pyramid YOLO7 algorithm comprises the following steps:

obtaining an original input image with a resolution of w × h pixels captured by the camera;

performing rectangular window cropping on keyframes of the original input image, and denoting a starting point and an ending point of the rectangular window cropping as $A(x_1,y_1)$ and B $(x_2,y_2)$;

acquiring the captured images once every 10 ms, and determining whether an image threshold is greater than 5 ml/h; acquiring an image of which keyframes collected by the camera at a current moment are subjected to the rectangular window cropping and denoting as Image A when the image threshold is greater than 5 ml/h and a current injected amount is less than half of a capacity of the refill;

taking the current moment as the starting point to acquire images of which keyframes over the previous 500 ms and the previous 2 s are subjected to the rectangular window cropping, and denoting as Images B and C, respectively;

processing Images A, B and C respectively according to a preset target detection model to obtain target detection probabilities $P_A$, $P_B$ and $P_C$ of Images A, B and C;

obtaining maximum values of all dimensions of the target detection probabilities $P_A$, $P_B$ and $P_C$, and obtaining an injection speed and an injection amount at the current moment according to the obtained maximum values and a maximum value determination principle; and

starting a new round of detection after completion of the current detection.

9. The intelligent control method for a refill-type screw insulin injection pump according to claim 8, **characterized in that** the starting point A $(x_1,y_1)$ and the ending point B $(x_2,y_2)$ of the rectangular window cropping are determined according to the following methods:

(1) historically detected data is analyzed to obtain coordinates of a maximum circumscribed rectangle area of a target object hotspot marked by the pyramid YOLO7 algorithm with a self-adaptive weight over the most recent 7 days, which are respectively denoted as: $S_1\{(x_{11},y_{11}), (x_{21},y_{21})\}$, $S_2\{(x_{12},y_{12}), (x_{22},y_{22})\}$, $S_3\{(x_{13},y_{13}), (x_{23},y_{23})\}$ ...... $S_n\{(x_{1n},y_{1n}), (x_{2n},y_{2n})\}$;

coordinates of an X direction and a Y direction of the starting point A $(x_1, y_1)$ are expressed as Formulae (1) and (2) below:

$$x_1 = 0.8 * (x'_{11} + x'_{12} + x'_{13} + \cdots + x'_{1[n/2]})/[n/2] \qquad (1);$$

$$y_1 = 0.8 * (y'_{11} + y'_{12} + y'_{13} + \cdots + y'_{1[n/2]})/[n/2] \qquad (2);$$

in the formulae, [n/2] is n/2 rounded down, and $x_1$, $y_1$ are both greater than and equal to 1; $x'_{11}$, $x'_{12}$, $x'_{13} \ldots \ldots x'_{1[n/2]}$, and $y'_{11}, y'_{12}, y'_{13} \ldots \ldots y'_{1[n/2]}$ are data series $x_{11}, x_{12}, x_{13}, \ldots \ldots x_{1n}$ and $y_{11}, y_{12}, y_{13}, \ldots \ldots y_{1n}$ are arranged in ascending order respectively, and the first 50% of the data series are taken;
coordinates of an X direction and a Y direction of the ending point B $(x_2, y_2)$ are expressed as Formulae (3) and (4) below:

$$x_2 = 0.8 * (x'_{21} + x'_{22} + x'_{23} + \cdots + x'_{2[n/2]})/[n/2] \qquad (3);$$

$$y_2 = 0.8 * (y'_{21} + y'_{22} + y'_{23} + \cdots + y'_{2[n/2]})/[n/2] \qquad (4);$$

in the formulae, $x_2$ is less than and equals to 1280, $y_2$ is less than and equals to 720; $x'_{21}, x'_{22}, x'_{23} \ldots x'_{2[n/2]}$ and $y'_{21}$, $y'_{22}, y'_{23} \ldots y'_{2[n/2]}$ are data series $x_{21}, x_{22}, x_{23}, \ldots x_{2n}$ and $y_{21}, y_{22}, y_{23}, \ldots y_{2n}$ are arranged in ascending order, and the first 50% of the data series are taken;

(2) when the cumulative number of days of historically data is less than 7 days, coordinates of the starting point A are set as (1, 1), and coordinates of the ending point B are set as (1280, 720).

10. The intelligent control method for a refill-type screw insulin injection pump according to claim 9, **characterized in that** Image A is processed according to the preset target detection model, **characterized by** comprising the following steps:

performing target object detection on Image A, obtaining a maximum probability that the injection amount is less than or equals to 0, is greater than 0 but less than 1/2 and equals to 1 in Image A, and denoting as P{a, b, c};
dividing Image A by two equal parts into four sub-images in length and width directions, obtaining maximum probabilities that the injection amount is less than or equals to 0, is greater than 0 but less than 1/2 and equals to 1 in the current sub-images, and denoting as $P_1$, $P_2$, $P_3$ and $P_4$; and selecting maximum values of $P_1$, $P_2$, $P_3$ and $P_4$ of the four sub-images in each dimension, and denoting as Ps{d, e, f};
dividing Image A by four equal parts into sixteen sub-images in length and width directions, obtaining maximum probabilities that the injection amount is less than or equals to 0, is greater than 0 but less than 1/2 and equals to 1 in the current sub-images and denoting as $P_{11} \ldots P_{14} \ldots P_{24} \ldots P_{44}$; and selecting maximum values of $P_{11} \ldots P_{14} \ldots P_{24} \ldots P_{44}$ of the sixteen sub-images in each dimension and denoting as $P_{55}${g, h, i};
obtaining the target detecting probability $P_A$ of Image A according to Ps{d, e, f}, $P_{55}${g, h, i} and Formula (5):

$$P_A = \left\{ \frac{1}{3} \times (w_1 \times a + w_2 \times d + w_3 \times g), \frac{1}{3} \times (w_4 \times b + w_5 \times e + w_6 \times h), \frac{1}{3} \times (w_7 \times c + w_8 \times f + w_9 \times i) \right\}$$

$$(5);$$

in the formula, $w_1 \ldots \ldots w_9$ are weights and are obtained by the following formulae:

$$w_1 = \frac{a}{a+b+c}, \quad w_2 = \frac{d}{d+e+f}, \quad w_3 = \frac{g}{g+h+i} \qquad (6);$$

$$w_4 = \frac{b}{a+b+c}, \quad w_5 = \frac{e}{d+e+f}, \quad w_6 = \frac{h}{g+h+i} \qquad (7);$$

$$W_7 = \frac{c}{a+b+c}, \quad W_8 = \frac{f}{d+e+f}, \quad W_9 = \frac{i}{g+h+i} \qquad (8)$$

11. The intelligent control method for a refill-type screw insulin injection pump according to claim 10, **characterized in that** the same steps are taken to process Images B and C to obtain the target detecting probabilities $P_B$ and Pc.

$$W_7 = \frac{c}{a+b+c}, \quad W_8 = \frac{f}{d+e+f}, \quad W_9 = \frac{i}{g+h+i} \qquad (8)$$

FIG. 1

**FIG. 1**

**FIG. 2**

10

7

30

28

16

27

26

15

17

**FIG. 3**

**FIG. 4**

**FIG. 5**

FIG. 6

26    19    38

17

B

**FIG. 7**

A $(x_1, y_1)$

Refill piston

$(x_2, y_2)$
B

$h$

$w$

**FIG. 8**

A

→ P{a,b,c}

| $A_1$ | $A_2$ |
|---|---|
| $A_3$ | $A_4$ |

→ $(P_1, P_2, P_3, P_4)$

| $A_{11}$ | $A_{12}$ | $A_{13}$ | $A_{14}$ |
|---|---|---|---|
| $A_{21}$ | $A_{22}$ | $A_{23}$ | $A_{24}$ |
| $A_{31}$ | $A_{32}$ | $A_{33}$ | $A_{34}$ |
| $A_{41}$ | $A_{42}$ | $A_{43}$ | $A_{44}$ |

→ $(P_{11}, P_{12}, ..., P_{41})$

**FIG. 9**

FIG. 10

FIG. 11

Europäisches
Patentamt
European
Patent Office
Office européen
des brevets

# EUROPEAN SEARCH REPORT

Application Number

EP 24 18 6419

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 921 038 A (SHENZHEN RUIYU MEDICAL TECH CO LTD) 13 November 2020 (2020-11-13) | 1,2 | INV. A61M5/142 |
| A | * the whole document * | 3-11 | A61M5/168 A61M5/172 |
| | ----- | | G16H20/17 |
| A | US 10 850 032 B2 (YPSOMED AG [CH]) 1 December 2020 (2020-12-01) * figures 2a-13 * | 1-11 | |
| | ----- | | |

TECHNICAL FIELDS
SEARCHED          (IPC)

A61M
G16H

The present search report has been drawn up for all claims

| Place of search | Date of completion of the search | Examiner |
|---|---|---|
| Munich | 3 December 2024 | Haslinde, Verena |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...............................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 18 6419

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2024

| Patent document cited in search report | | Publication date | Patent family member(s) | | Publication date |
|---|---|---|---|---|---|
| CN 111921038 | A | 13-11-2020 | NONE | | |
| US 10850032 | B2 | 01-12-2020 | CN | 106029125 A | 12-10-2016 |
| | | | DK | 3110475 T3 | 23-09-2019 |
| | | | EP | 3110475 A1 | 04-01-2017 |
| | | | ES | 2745467 T3 | 02-03-2020 |
| | | | PL | 3110475 T3 | 31-12-2019 |
| | | | US | 2016361494 A1 | 15-12-2016 |
| | | | US | 2021038810 A1 | 11-02-2021 |
| | | | US | 2024299654 A1 | 12-09-2024 |
| | | | WO | 2015127965 A1 | 03-09-2015 |

EPO FORM P0459

**REFERENCES CITED IN THE DESCRIPTION**

**Patent documents cited in the description**

- CN 108159530 A

- CN 109529154 A